# EUROPEAN PATENT APPLICATION

(11) **EP 1 468 990 A1**
(43) Date of publication of application: **20.10.2004**
(21) Application number: 02805485.6
(22) Date of filing: 19.12.2002
(51) Int. Cl.: C07D 209/48, C07D 211/26, C07D 231/12, C07D 233/70, C07D 233/84, C07D 239/26, C07D 239/42, C07D 261/08, C07D 263/32, C07D 277/40, C07D 277/56, C07D 295/12, C07D 295/14, C07D 307/91, C07D 313/18, A61K 31/496, A61K 31/517, A61P 3/04, A61P 25/22, A61P 25/24, A61P 43/00

(54) **PIPERAZINE DERIVATIVE**

(30) Priority: 21.12.2001 JP 2001389419
(71) Applicant: Taisho Pharmaceutical Co. Ltd., Tokyo 171-8633 (JP)
(72) Inventor: NAKAZATO, Atsuro, Taisho Pharmaceutical Co., Ltd., Tokyo 170-8633 (JP); ISHII, Takaaki, Taisho Pharmaceutical Co., Ltd., Tokyo 170-8633 (JP); NOZAWA, Dai, c/o Taisho Pharmaceutical Co., Ltd., Tokyo 170-8633 (JP)
(74) Representative: HOFFMANN EITLE
(86) International application number: PCT/JP2002/013317
(87) International publication number: WO 2003/053927

(57) **Abstract**

A piperazine derivative represented by the formula (1): 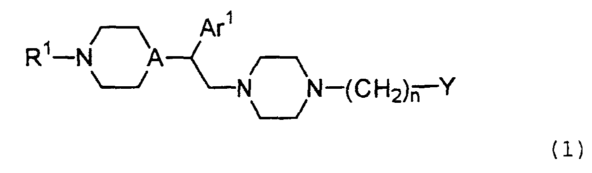 wherein n is an integer of 1 to 8; R¹ represents hydrogen or C₁₋₁₀ alkyl; A represents CH or nitrogen; Ar¹ represents phenyl or substituted phenyl; and Y represents a group represented by the formula Y¹-Y²-Ar² or Y³-Y⁴(Ar⁵)-Ar⁶ or a pharmaceutically acceptable salt of the derivative. The novel piperazine derivative has MC4 receptor antagonistic activity.

## Description

### Technical Field

This invention relates to a therapeutic agent for anxiety neurosis or depression which comprises as an active ingredient, an MC4 receptor antagonist and to a novel piperazine derivative having MC4 receptor antagonistic activity.

### Background Art

Recent advances in pathologic physiology suggest that stress is deeply involved in the onset mechanism of anxiety neurosis and depression. Dysfunction of the neuroendocrine system, the representative of which is the dysfunction of the hypothalamus-hypophysis-adrenal system, is known as an intracerebral reaction caused by stress. With this background, neuropeptides have recently attracted attention as the cause for the onset of depression or anxiety which are found in the hypothalamus and which affect the neuroendocrine system.

Such neuropeptides include corticotropin-releasing factor (CRF), pro-opiomelanocortin (POMC) and the like. Melanocortins produced from POMC [adrenocorticotropic hormone (ACTH) and melanin cell stimulating hormone (MSH)] are major neuropeptides in the hypothalamus; however, it has not yet been reported that substances acting on the melanocortin receptors are involved in stress reaction as well as in depression and anxiety neurosis.

The melanocortin receptors are classified into five subtypes of MC1 to MC5. Among these subtypes, selective agonists and antagonists of the peptide type have been reported for the melanocortin receptor subtype MC4, but no reports have been made on agonists and antagonists of the non-peptide type.

It is the object of this invention to provide novel compounds having antagonistic activity against the melanocortin receptor subtype MC4.

### Disclosure of Invention

The present inventors made their intensive and diligent studies on the relationship of the melanocortin receptor subtypes with depression and anxiety neurosis and with stress reaction as well as on novel piperazine derivatives. Consequently, it was discovered that certain piperazine derivatives had excellent MC4 receptor antagonistic activity, upon which this invention has been completed.

This invention will be described below.

This invention relates to a piperazine derivative represented by the formula (1): 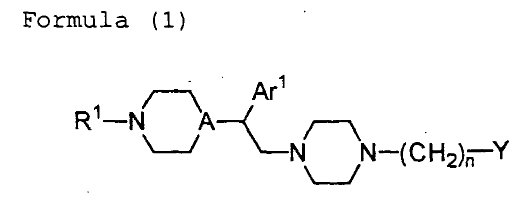 wherein n represents an integer of 1 to 8; R¹ represents a hydrogen atom or a C₁₋₁₀ alkyl group; A represents CH or a nitrogen atom; Ar¹ represents a phenyl group, or a phenyl group substituted with 1 to 3 groups arbitrarily selected from a C₁₋₁₀ alkyl group, a C₁₋₁₀ alkoxy group, an aralkyloxy group, a hydroxyl group, a halogen atom, a nitro group, an amino group, a mono- or di-substituted amino group with a C₁₋₆ alkyl group(s), a trifluoromethyl group, a trifluoromethoxy group, a cyano group, a carbamoyl group or a phenyl group; and Y is a group represented by the formula Y¹-Y²-Ar² wherein Y¹-Y² represents a single bond, an oxygen atom, C(=O), CH=CH, C(=O)-N(R²) or N(R²)-C(=O) (wherein R² represents a hydrogen atom or a C₁₋₁₀ alkyl group); and Ar² represents a phthalimido-1-yl group, a dibenzofuranyl group, a C₃₋₁₀ cycloalkyl group, a C₂₋₉ oxacycloalkyl group, a C₂₋₉ lactam-1-yl group, a 1H-quinazoline-2,4-dion-1-yl group, or a group represented by the following formula: 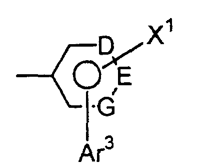 wherein D, E and G may be the same or different and each represents CH or a nitrogen atom; X¹ represents a hydrogen atom, a halogen atom, a C₁₋₁₀ alkyl group, a C₁₋₁₀ alkoxy group, a hydroxyl group, an amino group, a carbamoyl group, a C₁₋₅ alkylthio group or a phenyl group; and Ar³ represents a phenyl group, a naphthyl group, a phenoxy group, or alternatively, a phenyl, naphthyl or phenoxy group substituted with 1 to 3 groups arbitrarily selected from a C₁₋₁₀ alkyl group, a C₁₋₁₀ alkoxy group, an aralkyloxy group, a hydroxyl group, a halogen atom, a nitro group, an amino group, a mono- or di-substituted amino group with a C₁₋₅ alkyl group(s), a trifluoromethyl group, a trifluoromethoxy group, a cyano group, a carbamoyl group or a phenyl group, or a group represented by the following formula: 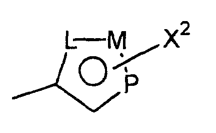 wherein L, M and P may be the same or different and each represents CH, NH, a nitrogen atom, an oxygen atom or a sulfur atom; and X² represents a hydrogen atom, a halogen atom, a C₁₋₁₀ alkyl group, a C₁₋₁₀ alkoxy group, a hydroxyl group, an amino group, a carbamoyl group, a C₁₋₅ alkylthio group or a phenyl group, or a group represented by the following formula: 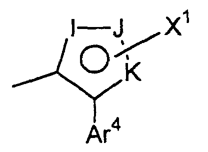 wherein I, J and K may be the same or different and each represents CH, NH, a nitrogen atom, an oxygen atom or a sulfur atom; X¹ is as previously defined; and Ar⁴ represents a phenyl group or a phenyl group substituted with 1 to 3 groups arbitrarily selected from a C₁₋₁₀ alkyl group, a C₁₋₁₀ alkoxy group, an aralkyloxy group, a hydroxyl group, a halogen atom, a nitro group, an amino group, a mono- or di-substituted amino group with a C₁₋₅ alkyl group(s), a trifluoromethyl group, a trifluoromethoxy group, a cyano group, a carbamoyl group or a phenyl group, or a group represented by the following formula: 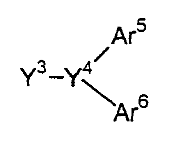 wherein Y³-Y⁴ represents CH₂-C(R^{a}) [wherein R^{a} represents a hydrogen atom or a group represented by the formula CO₂R^{b} or the formula CON(R^{b})R^{c} (wherein R^{b} and R^{c} may be the same or different and each represents a hydrogen atom or a C₁₋₁₀ alkyl group)], CH=C or C(=O)-CH; and Ar⁵ and Ar⁶ may be the same or different and each represents a phenyl group or a phenyl group substituted with 1 to 3 groups arbitrarily selected from a C₁₋₁₀ alkyl group, a C₁₋₁₀ alkoxy group, an aralkyloxy group, a hydroxyl group, a halogen atom, a nitro group, an amino group, a mono- or di-substituted amino group with a C₁₋₅ alkyl group(s), a trifluoromethyl group, a trifluoromethoxy group, a cyano group, a carbamoyl group or a phenyl group, or a group forming together with the adjacent carbon atom, a group represented by the following formula: 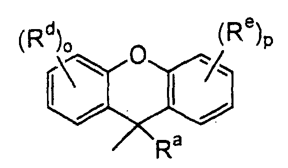 wherein R^{d} and R^{e} each represent a group arbitrarily selected from a hydrogen atom, a C₁₋₁₀ alkyl group, a C₁₋₁₀ alkoxy group, an aralkyloxy group, a hydroxyl group, a halogen atom, a nitro group, an amino group, a mono- or di-substituted amino group with a C₁₋₅ alkyl group(s), a trifluoromethyl group, a trifluoromethoxy group, a cyano group, a carbamoyl group or a phenyl group; R^{a} is as previously defined; and o and p each are an integer of 1 to 3,
or a pharmaceutically acceptable salt thereof. There may be stereoisomers and optical isomers of the piperazine derivatives of this invention, which are also embraced by this invention.

According to this invention, for the phenyl group substituted with 1 to 3 groups arbitrarily selected from a C₁₋₁₀ alkyl group, a C₁₋₁₀ alkoxy group, an aralkyloxy group, a hydroxyl group, a halogen atom, a nitro group, an amino group, a mono- or di-substituted amino group with a C₁₋₆ alkyl group(s), a trifluoromethyl group, a trifluoromethoxy group, a cyano group, a carbamoyl group or a phenyl group, there may, for example, be mentioned a 2-methylphenyl group, a 3-methylphenyl group, a 4-methylphenyl group, a 2-ethylphenyl group, a 3-ethylphenyl group, a 4-ethylphenyl group, a 2-propylphenyl group, a 3-propylphenyl group, a 4-propylphenyl group, a 4-isopropylphenyl group, a 4-*tert*-butylphenyl group, a 2-methoxyphenyl group, a 3-methoxyphenyl group, a 4-methoxylphenyl group, a 4-ethoxylphenyl group, a 4-isopropoxyphenyl group, a 4-benzyloxyphenyl group, a 4-hydroxyphenyl group, a 2-fluorophenyl group, a 3-fluorophenyl group, a 4-fluorophenyl group, a 2,4-difluorophenyl group, a 3,4-difluorophenyl group, a 3,5-difluorophenyl group, a 2-chlorophenyl group, a 3-chlorophenyl group, a 4-chlorophenyl group, a 2-bromophenyl group, a 3-bromophenyl group, a 4-bromophenyl group, a 4-nitrophenyl group, a 4-aminophenyl group, a 4-dimethylaminophenyl group, a 4-trifluoromethylphenyl group, a 4-trifluoromethoxyphenyl group, a 4-cyanophenyl group, a 4-carbamoylphenyl group, a 4-biphenyl group, etc.

For the phenyl group substituted with 1 to 3 halogen atoms, there may, for example, be mentioned a 2-fluorophenyl group, a 3-fluorophenyl group, a 4-fluorophenyl group, a 2,4-difluorophenyl group, a 3,4-difluorophenyl group, a 3,5-difluorophenyl group, a 2,4,6-trifluorophenyl group, a 2-chlorophenyl group, a 3-chlorophenyl group, a 4-chlorophenyl group, a 2-bromophenyl group, a 3-bromophenyl group, a 4-bromophenyl group, etc.

For the phenyl group substituted with 1 to 3 groups arbitrarily selected from a C₁₋₁₀ alkyl group, C₁₋₁₀ alkoxy group, a halogen atom, a nitro group, an amino group, a mono- or di-substituted amino group with a C₁₋₆ alkyl group(s), a trifluoromethyl group, a trifluoromethoxy group, a cyano group, a carbamoyl group or a phenyl group, there may, for example, be mentioned a 2-methylphenyl group, a 3-methylphenyl group, a 4-methylphenyl group, a 2-ethylphenyl group, a 3-ethylphenyl group, a 4-ethylphenyl group, a 2-propylphenyl group, a 3-propylphenyl group, a 4-propylphenyl group, a 4-isopropylphenyl group, a 4-*tert*-butylphenyl group, a 2-methoxyphenyl group, a 3-methoxyphenyl group, a 4-methoxylphenyl group, a 4-ethoxylphenyl group, a 4-isopropoxyphenyl group, a 2-fluorophenyl group, a 3-fluorophenyl group, a 4-fluorophenyl group, a 2,4-difluorophenyl group, a 3,4-difluorophenyl group, a 3,5-difluorophenyl group, a 2-chlorophenyl group, a 3-chlorophenyl group, a 4-chlorophenyl group, a 2-bromophenyl group, a 3-bromophenyl group, a 4-bromophenyl group, a 4-nitrophenyl group, a 4-aminophenyl group, a 4-dimethylaminophenyl group, a 4-trifluoromethylphenyl group, a 4-trifluoromethoxyphenyl group, a 4-cyanophenyl group, a 4-carbamoylphenyl group, a 4-biphenyl group, etc.

For the phenyl group substituted with 1 to 3 groups arbitrarily selected from a C₁₋₁₀ alkyl group, a C₁₋₁₀ alkoxy group, a halogen atom, a trifluoromethyl group, a trifluoromethoxy group or a carbamoyl group, there may, for example, be mentioned a 2-methylphenyl group, a 3-methylphenyl group, a 4-methylphenyl group, a 2-ethylphenyl group, a 3-ethylphenyl group, a 4-ethylphenyl group, a 2-propylphenyl group, a 3-propylphenyl group, a 4-propylphenyl group, a 4-isopropylphenyl group, a 4-*tert*-butylphenyl group, a 2-methoxyphenyl group, a 3-methoxyphenyl group, a 4-methoxylphenyl group, a 4-ethoxylphenyl group, a 4-isopropoxyphenyl group, a 2-fluorophenyl group, a 3-fluorophenyl group, a 4-fluorophenyl group, a 2,4-difluorophenyl group, a 3,4-difluorophenyl group, a 3,5-difluorophenyl group, a 2-chlorophenyl group, a 3-chlorophenyl group, a 4-chlorophenyl group, a 2-bromophenyl group, a 3-bromophenyl group, a 4-bromophenyl group, a 4-trifluoromethylphenyl group, a 4-trifluoromethoxyphenyl group, a 4-carbamoylphenyl group, etc.

For the naphthyl group substituted with 1 to 3 groups arbitrarily selected from a C₁₋₁₀ alkyl group, a C₁₋₁₀ alkoxy group, an aralkyloxy group, a hydroxyl group, a halogen group, a nitro group, an amino group, a mono- or di-substituted amino group with a C₁₋₆ alkyl group(s), a trifluoromethyl group, a trifluoromethoxy group, a carbamoyl group or a phenyl group, there may, for example, be mentioned a 2-methylnaphthalen-1-yl group, a 3-methylnaphthalen-1-yl group, a 4-methylnaphthalen-1-yl group, a 2-ethylnaphthalen-1-yl group, a 3-ethylnaphthalen-1-yl group, a 4-ethylnaphthalen-1-yl group, a 2-propylnaphthalen-1-yl group, a 3-propylnaphthalen-1-yl group, a 4-propylnaphthalen-1-yl group, a 2-methoxynaphthalen-1-yl group, a 3-methoxynaphthalen-1-yl group, a 4-methoxynaphthalen-1-yl group, a 6-methoxynaphthalen-1-yl group, a 4-ethoxynaphthalen-1-yl group, a 4-isopropoxynaphthalen-1-yl group, a 4-benzyloxynaphthalen-1-yl group, a 4-hydroxynaphthalen-1-yl group, a 2-fluoronaphthalen-1-yl group, a 3-fluoronaphthalen-1-yl group, a 4-fluoronaphthalen-1-yl group, a 2-chloronaphthalen-1-yl group, a 3-chloronaphthalen-1-yl group, a 4-chloronaphthalen-1-yl group, a 2-bromonaphthalen-1-yl group, a 3-bromonaphthalen-1-yl group, a 4-bromonaphthalen-1-yl group, a 4-nitronaphthalen-1-yl group, a 4-aminonaphthalen-1-yl group, a 4-dimethyaminonaphthalen-1-yl group, a 4-trifluoromethylnaphthalen-1-yl group, a 4-trifluoromethoxynaphthalen-1-yl group, a 4-cyanonaphthalen-1-yl group, a 4-carbamoylnaphthalen-1-yl group, a 4-phenylnaphthalen-1-yl group, etc.

For the phenoxy group substituted with 1 to 3 groups arbitrarily selected from a C₁₋₁₀ alkyl group, a C₁₋₁₀ alkoxy group, an aralkyloxy group, a hydroxyl group, a halogen group, a nitro group, an amino group, a mono- or di-substituted amino group with a C₁₋₆ alkyl group(s), a trifluoromethyl group, a trifluoromethoxy group, a cyano group, a carbamoyl group or a phenyl group, there may, for example, be mentioned a 2-methylphenoxy group, a 3-methylphenoxy group, a 4-methylphenoxy group, a 2-ethylphenoxy group, a 3-ethylphenoxy group, a 4-ethylphenoxy group, a 2-propylphenoxy group, a 3-propylphenoxy group, a 4-propylphenoxy group, a 2-methoxyphenoxy group, a 3-methoxyphenoxy group, a 4-methoxylphenoxy group, a 4-ethoxylphenoxy group, a 4-isopropoxyphenoxy group, a 4-benzyloxyphenoxy group, a 4-hydroxyphenoxy group, a 2-fluorophenoxy group, a 3-fluorophenoxy group, a 4-fluorophenoxy group, a 2-chlorophenoxy group, a 3-chlorophenoxy group, a 4-chlorophenoxy group, a 2-bromophenoxy group, a 3-bromophenoxy group, a 4-bromophenoxy group, a 4-nitrophenoxy group, a 4-aminophenoxy group, a 4-dimethylaminophenoxy group, a 4-trifluoromethylphenoxy group, a 4-trifluoromethoxyphenoxy group, a 4-cyanophenoxy group, a 4-carbamoylphenoxy group, a 4-phenylphenoxy group, etc.

The C₁₋₄ alkyl group and the C₁₋₁₀ alkyl group each refer to a straight- or branched-alkyl group. The C₁₋₄ alkyl group is, for example, a methyl group, an ethyl group, a propyl group, an isopropyl group, a butyl group, an isobutyl group, a *tert*-butyl group or the like. The C₄₋₁₀ alkyl group is, for example, a pentyl group, an isopentyl group, a 1-ethylpropyl group, a hexyl group, an isohexyl group, a 1-ethylbutyl group, a heptyl group, an isoheptyl group, an octyl group, a nonyl group, a decyl group or the like.

The C₁₋₁₀ alkoxy group refers to a straight- or branched-alkoxy group and is, for example, a methoxy group, an ethoxy group, a propoxy group, an isopropoxy group, a butoxy group, an isobutoxy group, a pentyloxy group, an isopentyloxy group, a hexyloxy group, a heptyloxy group, an octyloxy group, a nonyloxy group, a decyloxy group or the like.

The C₃₋₁₀ cycloalkyl group refers to a monocyclic or polycyclic cycloalkyl group and is, for example, a cyclopropyl group, a cyclobutyl group, a cyclopentyl group, a cyclohexyl group, a cycloheptyl group, a cyclooctyl group, a cyclononyl group, a cyclodecyl group, an adamantan-1-yl group, an adamantan-2-yl group or the like.

The C₂₋₉ oxacycloalkyl group refers to a cycloalkyl group wherein one of the ring carbon atoms is replaced by an oxygen atom and is, for example, an oxiranyl group, an oxetanyl group, a tetrahydrofuranyl group, a tetrahydropyranyl group, an oxepanyl group, an oxocanyl group, an oxonanyl group, an oxecanyl group or the like.

The C₁₋₅ alkylthio group refers to a straight- or branched-alkylthio group and is, for example, a methylthio group, an ethylthio group, a propylthio group, an isopropylthio group, a butylthio group, an isobutylthio group, a pentylthio group, an isopentylthio group or the like.

The mono- and di-substituted amino groups with a C₁₋₆ alkyl group(s) each refer to an amino group substituted with one or two straight- or branched-alkyl groups and are, for example, a methylamino group, an ethylamino group, a propylamino group, a dimethylamino group, a diethylamino group, a dipropylamino group or the like.

The halogen atom refers to a fluorine atom, a chlorine atom, a bromine atom or an iodine atom.

The pharmaceutically acceptable salts of this invention are, for example, salts with a mineral acid such as sulfuric acid, hydrochloric acid or phosphoric acid, or salts with an organic acid such as acetic acid, oxalic acid, lactic acid, tartaric acid, fumaric acid, maleic acid, methanesulfonic acid or benzenesulfonic acid.

The compounds of the formula (1) may be prepared according to General Preparation Processes 1 to 8 as described below. However, the methods for preparing the compounds of this invention are not to be limited to those processes.

In the reaction schemes below, Ar¹, Ar⁴, Ar⁵, Ar⁶, A, Y, D, E, G, I, J, K, X¹ and n are as previously defined, Y⁵ represents Y provided that a carbonyl group is excluded, X⁴ represents a chlorine atom, a bromine atom or an iodine atom, R⁴ represents a conventional amino protecting group such as an ethoxycarbonyl group or a benzyloxycarbonyl group, R⁵ represents a C₁₋₁₀ alkyl group, the group Boc represents a *tert*-butoxycarbonyl group, and the symbol* means it to be optically active.

### (General Preparation Process 1)

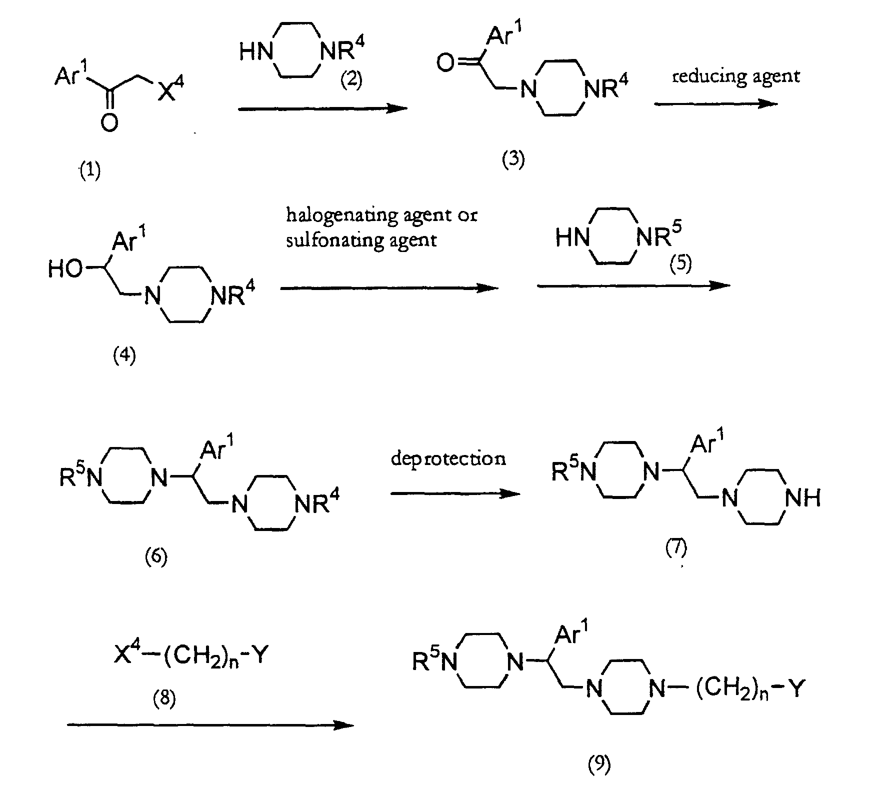

The compound (1) is allowed to react with the compound (2) in the presence or absence of a base in an inert solvent to form the compound (3) and then the carbonyl group may be reduced in an inert solvent to synthesize the compound (4). The compound (4) is allowed to react with a halogenating agent or a sulfonating agent such as an alkylsulfonyl halide or an arylsulfonyl halide in the presence or absence of a base in an inert solvent, whereby the hydroxyl group is converted to a suitable leaving group. The compound (6) may then be synthesized by reaction with the piperazine derivative (5) in the presence or absence of a base in an inert solvent. Subsequently, the compound (6) is subjected to the deprotection of the amino group to form the compound (7), and then reaction with the compound (8) in the presence or absence of a base in an inert solvent may produce the compound (9) of this invention.

The base as used herein refers to, for example, an organic amine such as triethylamine, diisopropylethylamine or pyridine, or an inorganic base such as potassium carbonate, sodium hydrogencarbonate, sodium hydroxide, potassium hydroxide or sodium hydride. The reduction refers to, for example, reduction under acidic, neutral or alkaline conditions using a boron reducing agent such as sodium borohydride, sodium cyanoborohydride, lithium borohydride, L-Selectride or K-Selectride, or an aluminum reducing agent such as lithium aluminum hydride, Red-A1 or diisobutyl aluminum hydride. The halogenating agent refers to, for example, a conventional halogenating agent for alcohol such as thionyl chloride, thionyl bromide or phosphoryl chloride. The sulfonating agent represented by an alkylsulfonyl halide or an arylsulfonyl halide refers to, for example, a conventional sulfonating agent for alcohol such as methanesulfonyl chloride, benzenesulfonyl chloride, toluenesulfonyl chloride or trifluoromethanesulfonyl chloride. The inert solvents are, for example, alcohols such as methanol and ethanol, ethers such as diethyl ether and tetrahydrofuran, hydrocarbons such as toluene and benzene, halogenated hydrocarbon solvents such as chloroform and dichloromethane, dimethylformamide, acetonitrile, water and a mixture of these solvents.

### (General Preparation Process 2)

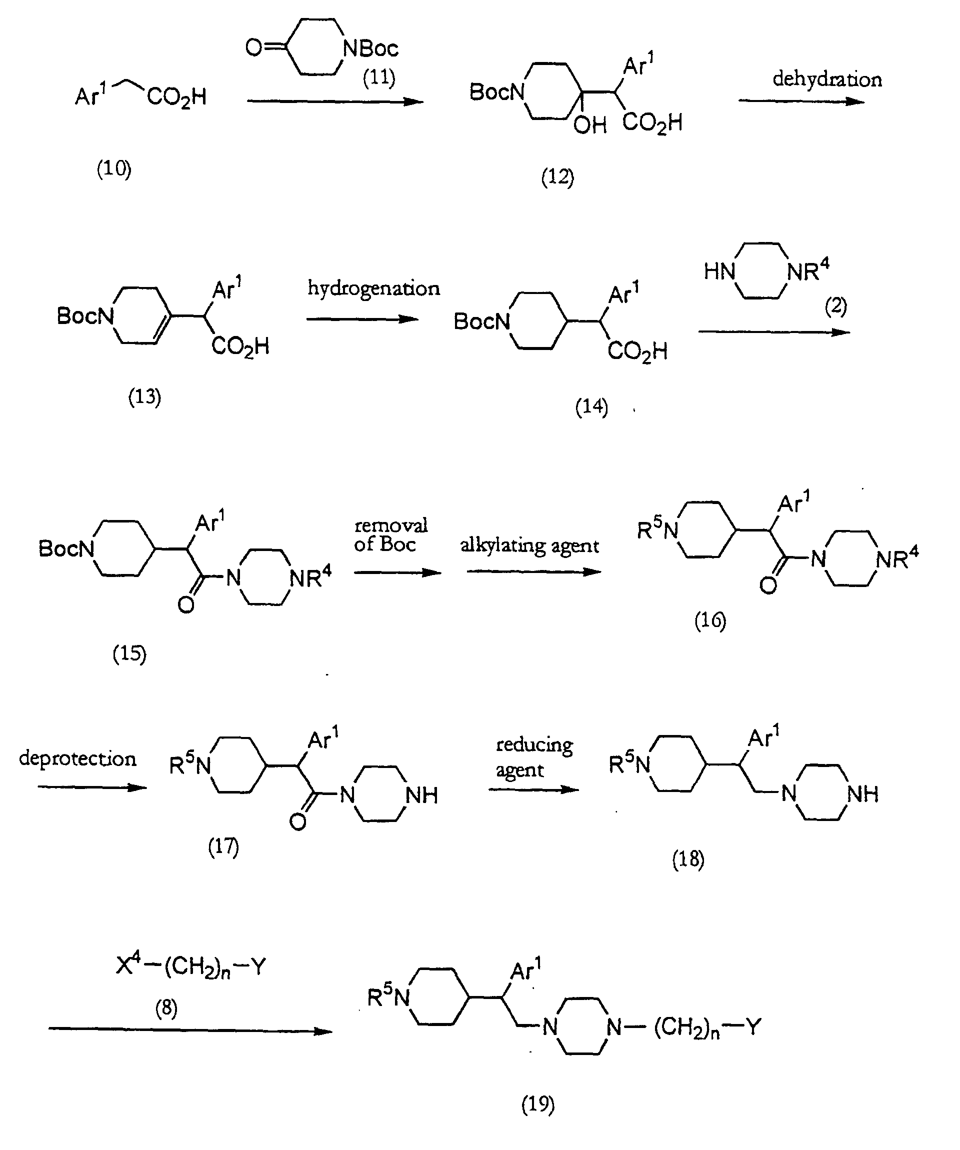

The compound (13) may be synthesized by treating the compound (10) with a base in an inert solvent, reacting it with the compound (11) to form the compound (12) and then treating the compound with an acid in an inert solvent. After the compound (13) is converted to the compound (14) by hydrogenation in an inert solvent, the latter compound is condensed with the compound (2) in an inert solvent to synthesize the compound (15). The Boc group of the compound (15) is deprotected in an inert solvent and is allowed to react with an alkylating agent in the presence or absence of a base in an inert solvent to perform conversion to the compound (16). The compound (17) may then be synthesized by deprotection of an amino group. After the compound (17) is converted to the compound (18) by reducing the amido group of the former compound in an inert solvent, the compound (19) of this invention may be obtained by reacting the compound (18) with the compound (8) in the presence or absence of a base in an inert solvent.

The bases as used herein are, for example, metal amides such as lithium diisopropylamide, lithium hexamethyldisilazide, sodium hexamethyldisilazide and potassium hexamethyldisilazide, metal hydrides such as sodium hydride and potassium hydride, organic amines such as triethylamine, diisopropylethylamine and pyridine, and inorganic bases such as potassium carbonate, sodium hydrogencarbonate, sodium hydroxide and potassium hydroxide. The acids as used herein are, for example, inorganic acids such as hydrochloric acid, hydrobromic acid, sulfuric acid, nitric acid and phosphoric acid, and organic acids such as p-toluenesulfonic acid, methanesulfonic acid, trifluoroacetic acid and formic acid. The hydrogenation as used herein refers to reaction using a metal catalyst commonly used such as palladium-carbon, palladium black, palladium hydroxide, platinum dioxide or Raney nickel in an inert solvent under hydrogen atmosphere. For the deprotection of an amino-protecting group such as Boc group, there may be used the method as described in Protective Groups in Organic Synthesis, by Theodora W. Greene and Peter G.M. Wuts. The alkylating agent as used herein refers to, for example, an alkyl halide such as methyl iodide, ethyl iodide, 1-bromopropane or 2-bromopropane or an alkyl sulfate such as dimethyl sulfate or diethyl sulfate. The reduction as used herein refers to, for example, reduction under acidic, neutral or basic conditions using a boron reducing agent such as diborane, or an aluminum reducing agent such as lithium aluminum hydride, Red-Al or diisobutyl aluminum hydride. The inert solvents as used herein are, for example, alcohols such as methanol and ethanol, ethers such as diethyl ether and tetrahydrofuran, hydrocarbons such as toluene and benzene, halogenated hydrocarbons such as chloroform and dichloromethane, dimethylformamide, acetonitrile, water and a mixture of these solvents.

### (General Preparation Process 3)

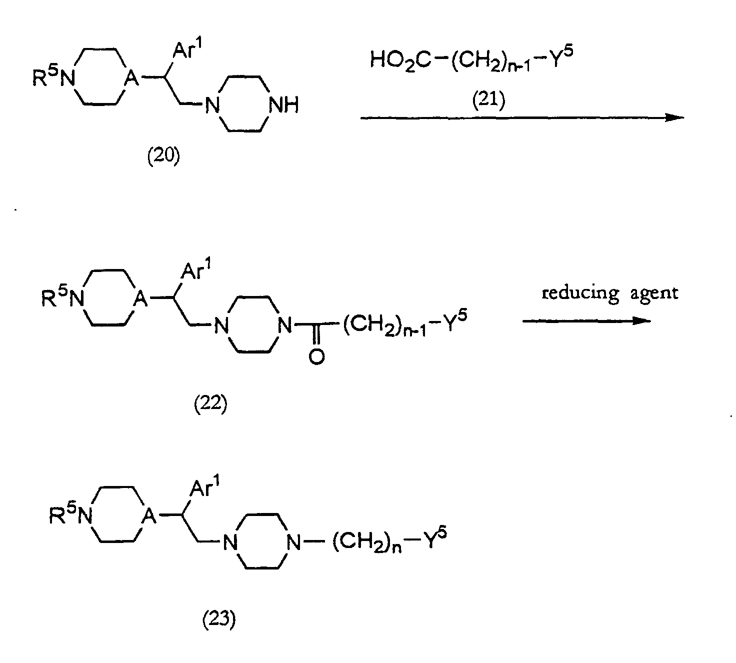

The compound (20) obtainable by General Preparation Process 1 or 2 is condensed with the compound (21) in an inert solvent to form the compound (22), and the amido group in the compound (22) is reduced in an inert solvent to prepare the compound (23) of this invention.

The condensation as used herein refers to, for example, amidation through an acid halide such as an acid chloride or acid bromide, amidation through a mixed anhydride using ethyl chlorocarbonate or isobutyl chlorocarbonate, or amidation using a condensing agent such as 1-(3,3-dimeythylaminopropyl)-3-ethylcarbodiimide, 1,3-dicyclohexylcarbodiimide, diphenylphosphorylazide, diethyl cyanophosphate or carbonyldiimidazole. The reduction as used herein refers to, for example, reduction under acidic, neutral or basic conditions using a boron reducing agent such as diborane, or an aluminum reducing agent such as lithium aluminum hydride, Red-Al or diisobutyl aluminum hydride. The inert solvents as used herein are, for example, alcohols such as methanol and ethanol, ethers such as diethyl ether and tetrahydrofuran, hydrocarbons such as toluene and benzene, halogenated hydrocarbons such as chloroform and dichloromethane, dimethylformamide, acetonitrile, water and a mixture of these solvents.

### (General Preparation Process 4)

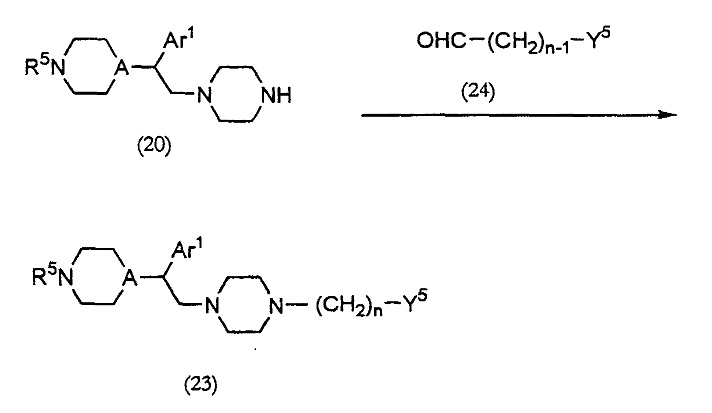

The compound (20) obtainable by General Preparation Process 1 or 2 and the compound (24) are treated with a reducing agent in the presence of an acid in an inert solvent to prepare the compound (23) of this invention.

The acids as used herein are, for example, inorganic acids such as hydrochloric acid, hydrobromic acid, sulfuric acid, nitric acid and phosphoric acid, and organic acids such as p-toluenesulfonic acid, methanesulfonic acid, trifluoroacetic acid, formic acid and acetic acid. The reducing agent as used herein refers to, for example, a boron reducing agent such as sodium borohydride, sodium cyanoborohydride, sodiumtriacetoxyborohydride or lithium borohydride. The inert solvents as used herein are, for example, alcohols such as methanol and ethanol, ethers such as diethyl ether and tetrahydrofuran, hydrocarbons such as toluene and benzene, halogenated hydrocarbons such as chloroform and dichloromethane, dimethylformamide, acetonitrile, water and a mixture of these solvents.

### (General Preparation Process 5)

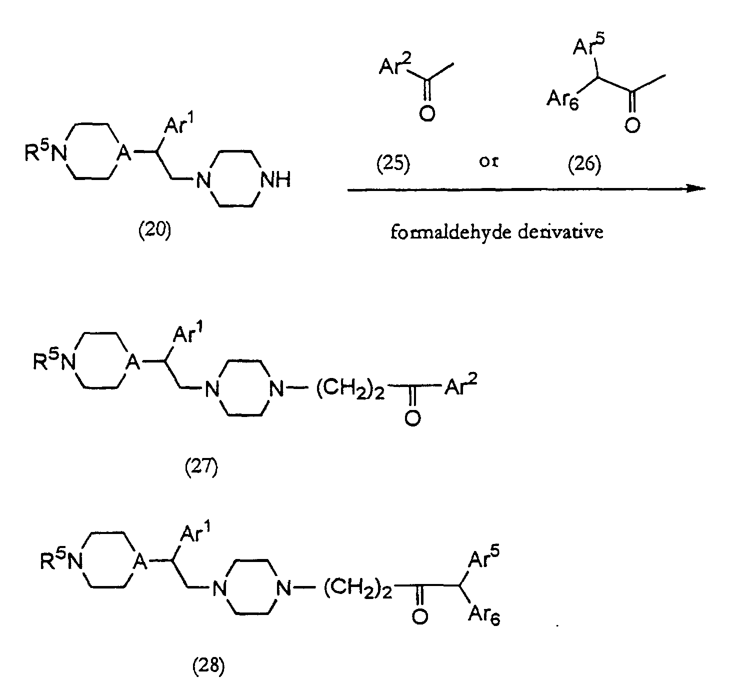

The compound (20) obtainable by General Preparation Process 1 or 2 and the compound (25) or the compound (26) are allowed to react with a formaldehyde derivative in the presence of an acid in an inert solvent to prepare the compound (27) or the compound (28) of this invention.

The formaldehyde derivative as used herein refers to formalin, paraformaldehyde, 1,3-dioxolan or the like. The acids as used herein are, for example, inorganic acids such as hydrochloric acid, hydrobromic acid, sulfuric acid, nitric acid and phosphoric acid, and organic acids such as p-toluenesulfonic acid, methanesulfonic acid, trifluoroacetic acid, formic acid and acetic acid. The inert solvents as used herein are, for example, alcohols such as methanol and ethanol, ethers such as diethyl ether and tetrahydrofuran, hydrocarbons such as toluene and benzene, halogenated hydrocarbons such as chloroform and dichloromethane, dimethylformamide, acetonitrile, water and a mixture of these solvents.

### (General Preparation Process 6)

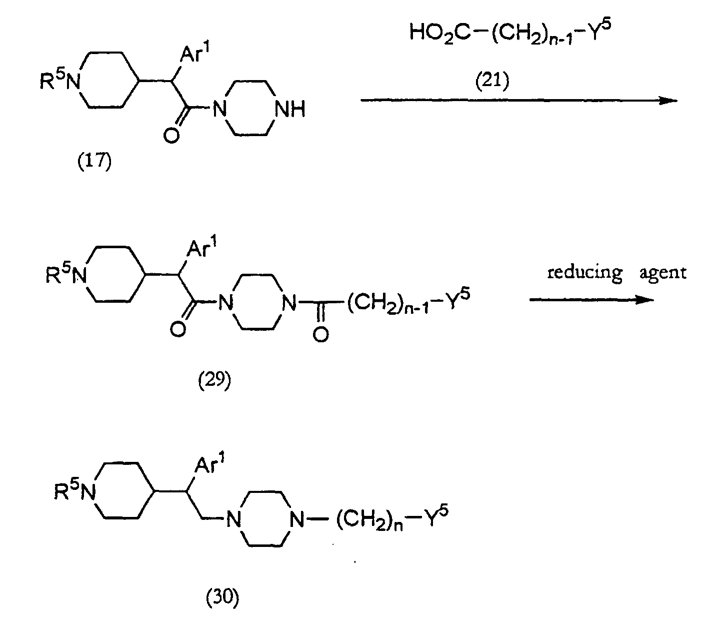

The compound (17) obtainable by General Preparation Process 2 is condensed with the compound (21) in an inert solvent to form the compound (29) and the amido group in the compound (29) is reduced in an inert solvent to prepare the compound (30) of this invention.

The condensation as used herein refers to, for example, amidation through an acid halide such as an acid chloride or acid bromide, amidation through a mixed anhydride using ethyl chlorocarbonate or isobutyl chlorocarbonate, or amidation using a condensing agent such as 1-(3,3-dimeythylaminopropyl)- 3-ethylcarbodiimide, 1,3-dicyclohexylcarbodiimide, diphenylphosphorylazide, diethyl cyanophosphate or carbonyldiimidazole. The reduction as used herein refers to, for example, reduction under acidic, neutral or basic conditions using a boron reducing agent such as diborane, or an aluminum reducing agent such as lithium aluminum hydride, Red-Al or diisobutyl aluminum hydride. The inert solvents as used herein are, for example, alcohols such as methanol and ethanol, ethers such as diethyl ether and tetrahydrofuran, hydrocarbons such as toluene and benzene, halogenated hydrocarbons such as chloroform and dichloromethane, dimethylformamide, acetonitrile, water and a mixture of these solvents.

### (General Preparation Process 7)

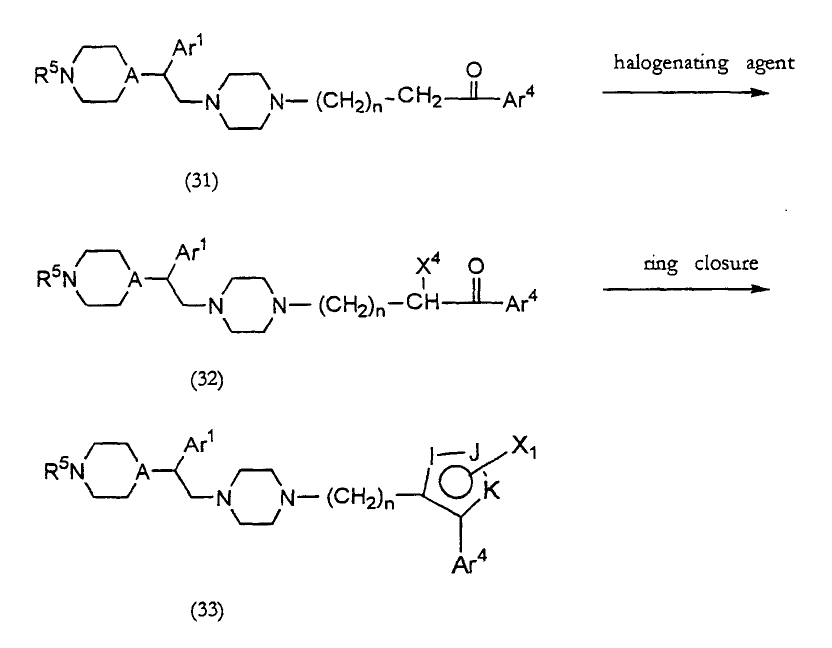

The compound (31) obtainable by General Preparation Process 1 or 2 is allowed to react with a halogenating agent in an inert solvent to form the compound (32), and the compound (32) is subjected to ring closure to prepare the compound (33) of this invention.

The halogenating agent as used herein refers to chlorine, bromine, iodine, N-chlorosuccinimide, N-bromosuccinimide, N-iodosuccinimide or the like. The ring closure refers to, for example, the formation of a heterocyclic ring by reaction with a reagent such as acetamide, urea, thiourea, acetamidine or phenylamidine in the presence or absence of a base. The bases as used herein are, for example, organic amines such as triethylamine, diisopropylamine and pyridine, and inorganic bases such as potassium carbonate, sodium hydrogencarbonate, sodium hydroxide, potassium hydroxide and sodium hydride. The inert solvents are, for example, alcohols such as methanol and ethanol, ethers such as diethyl ether and tetrahydrofuran, hydrocarbons such as toluene and benzene, halogenated hydrocarbon solvents such as chloroform and dichloromethane, dimethylformamide, acetonitrile, water and a mixture of these solvents.

### (General Preparation Process 8)

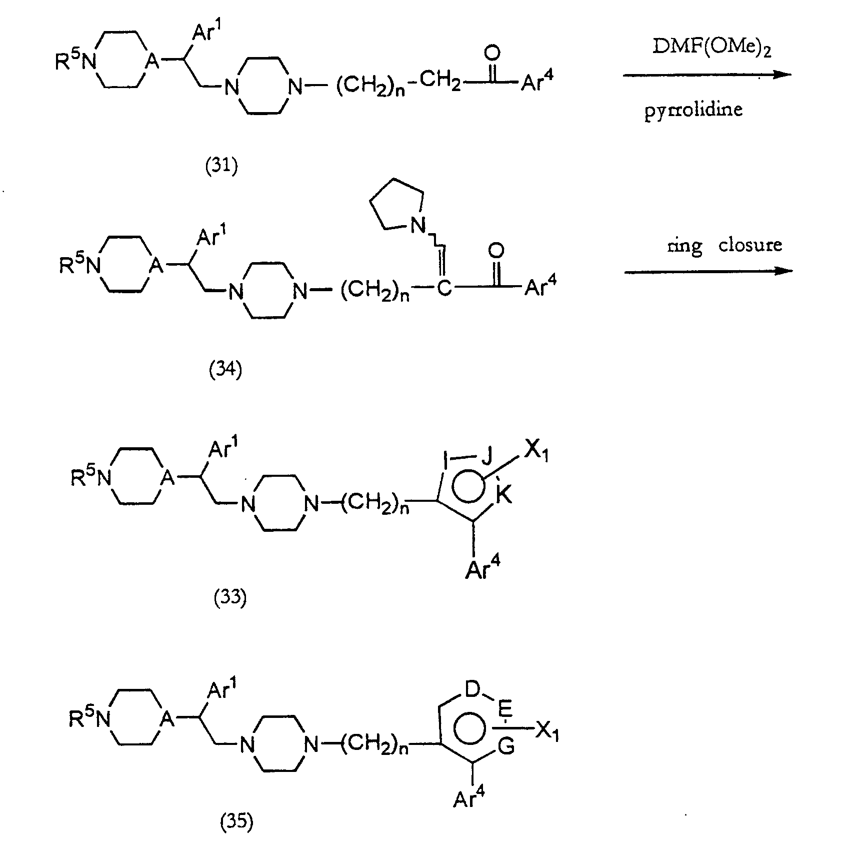

The compound (31) obtainable by General Preparation Process 1 or 2 is allowed to react with pyrrolidine and dimethylformamide dimethylacetal in an inert solvent to form the compound (34), and the compound (34) is subjected to ring closure to prepare the compound (33) of this invention or the compound (35) of this invention.

The ring closure as used herein refers to, for example, the formation of a heterocyclic ring by reaction with a reagent such as formamide, ammonium formate, urea, thiourea, guanidine or hydrazine in the presence or absence of a base. The bases as used herein are, for example, organic amines such as triethylamine, diisopropylethylamine and pyridine and inorganic bases such as potassium carbonate, sodium hydrogencarbonate, sodium hydroxide, potassium hydroxide and sodium hydride. The inert solvents are, for example, alcohols such as methanol and ethanol, ethers such as diethyl ether and tetrahydrofuran, hydrocarbons such as toluene and benzene, halogenated hydrocarbon solvents such as chloroform and dichloromethane, dimethylformamide, acetonitrile, water and a mixture of these solvents.

### (General Preparation Process 9)

The optically active compound (9), (19), (23), (27), (28), (30), (33) or (35) according to this invention may be obtained by resolving each racemate of the compound (9), (19), (23), (27), (28), (30), (33) or (35) according to this invention through the general optical resolution using an acidic chiral resolving agent or through the optical resolution with HPLC using a chiral stationary phase. Alternatively, the optically active compound (9) may be synthesized by resolving a racemate of the synthetic intermediate (6) or (7) through the optical resolution using an acidic chiral resolving agent or with HPLC using a chiral stationary phase, followed by the method described in General Preparation Process 1. Further, the optically active compound (19) may be synthesized by resolving a racemate of the synthetic intermediate (15), (16), (17) or (18) through the optical resolution using an acidic chiral resolving agent or with HPLC using a chiral stationary phase, followed by the method described in General Preparation Process 2. The optically active compound (23) may be synthesized by resolving a racemate of the synthetic intermediate (20) or (22) through the optical resolution using an acidic chiral resolving agent or with HPLC using a chiral stationary phase, followed by the method described in General Preparation Process 3 or 4. The optically active compound (27) or (28) may be synthesized by resolving a racemate of the synthetic intermediate (20) through the optical resolution using an acidic chiral resolving agent or with HPLC using a chiral stationary phase, followed by the method described in General Preparation Process 5. The optically active compound (30) may be synthesized by resolving a racemate of the synthetic intermediate (17) or (29) through the optical resolution using an acidic chiral resolving agent or with HPLC using a chiral stationary phase, followed by the method described in General Preparation Process 6. The optically active compound (33) may be synthesized by resolving a racemate of the synthetic intermediate (31) or (32) through the optical resolution using an acidic chiral resolving agent or with HPLC using a chiral stationary phase, followed by the method described in General Preparation Process 7 or 8. The optically active compound (35) may be synthesized by resolving a racemate of the synthetic intermediate (31) through the optical resolution using an acidic chiral resolving agent or with HPLC using a chiral stationary phase, followed by the method described in General Preparation Process 5.

The acidic chiral resolving agent as used herein refers to an optically active organic acid such as (+) or (-)-di-p-toluoyltartaric acid, (+) or (-)-dibenzoyltartaric acid, (+) or (-)-tartaric acid, (+) or (-)-mandelic acid, (+) or (-)-camphoric acid or (+) or (-)-camphorsulfonic acid.

The chiral stationary phase as used herein is a derivative such as a cellulose ester, a cellulose carbamate, an amylose carbamate, a crown ether or a polymetacrylate.

### (General Preparation Process 10)

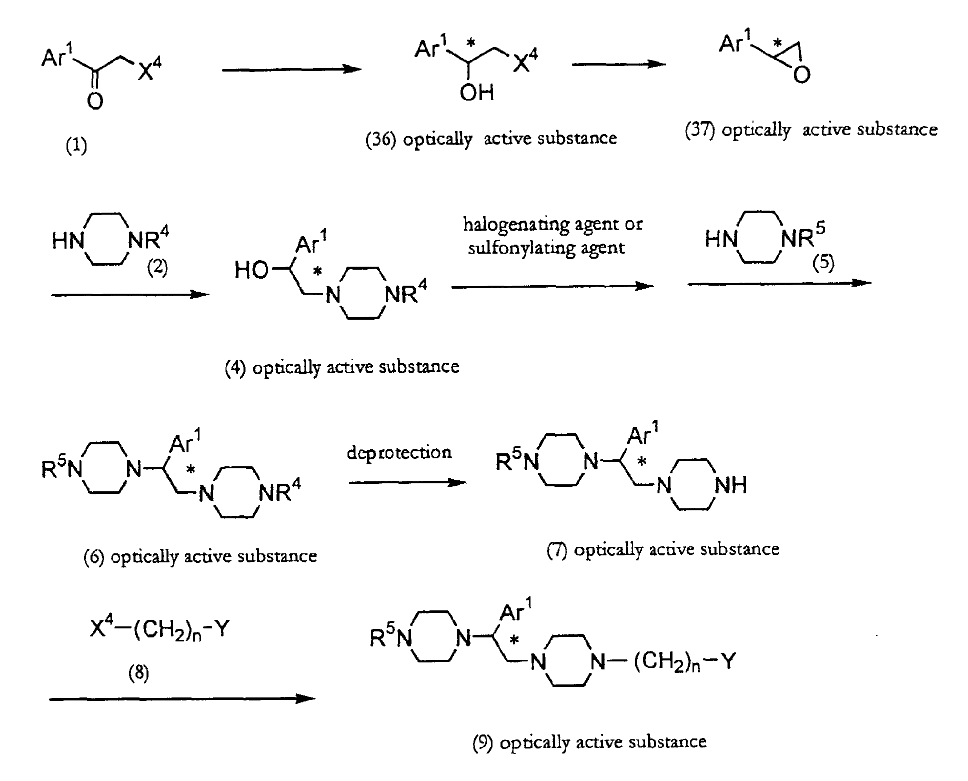

The optically active alcohol (36) may be obtained by asymmetric reduction of the compound (1) in an inert solvent. The optically active compound (4) may be synthesized by epoxidation of the compound (36) in the presence or absence of a base in an inert solvent, followed by reaction with the compound (2) in an inert solvent. Subsequently, the optically active compound (9) of this invention may be obtained from the optically active compound (4) in the same manner as the steps of preparing the compound (9) from the compound (4) as described in General Preparation Process 1.

The asymmetric reduction as used herein refers to reduction with a boran-tetrahydrofuran complex using as an asymmetric auxiliary group, an oxazaborolidine such as (R)-5,5-diphenyl-2-methyl-3,4-propano-1,3,2-oxazaborolidine or (S)-5,5-diphenyl-2-methyl-3,4-propano-1,3,2- oxazaborolidine, reduction using an optically active metal hydride such as (R)-B-3-pinanyl-9-boracyclo[3.3.1]nonane, (S)-B-3-pinanyl-9-boracyclo[3.3.1]nonane, (-)-chlorodiisopinocamphenylborane, (+)-chlorodiisopinocamphenylborane, (R,R)-2,5-dimethylborane, (S,S)-2,5-dimethylborane, (R)-BINAL-H or (S)-BINAL-H, or asymmetric hydrogenation using an optically active metal catalyst such as BINAL-luthenium complex. The bases as used herein are, for example, organic amines such as triethylamine, diisopropylethylamine and pyridine, inorganic bases such as potassium carbonate, sodium hydrogencarbonate, sodium hydroxide, potassium hydroxide and sodium hydride, metal amides such as lithium diisopropylamide, lithium hexamethyldisilazide, sodium hexamethyldisilazide and potassium hexamethyldisilazide and metal hydrides such as sodium hydride and potassium hydride. The inert solvents are, for example, alcohols such as methanol and ethanol, ethers such as diethyl ether and tetrahydrofuran, hydrocarbons such as toluene and benzene, halogenated hydrocarbon solvents such as chloroform and dichloromethane, dimethylformamide, acetonitrile, water and a mixture of these solvents.

### (General Preparation Process 11)

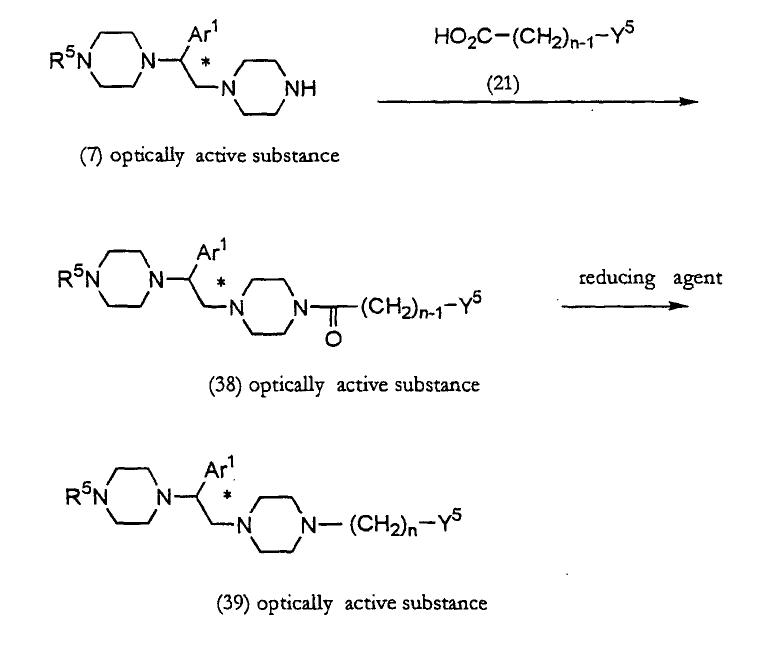

The optically active compound (39) of this invention may be obtained from the optically active compound (7) that can be prepared according to General Preparation Process 10 in the same manner as in the steps of General Preparation Process 3.

### (General Preparation Process 12)

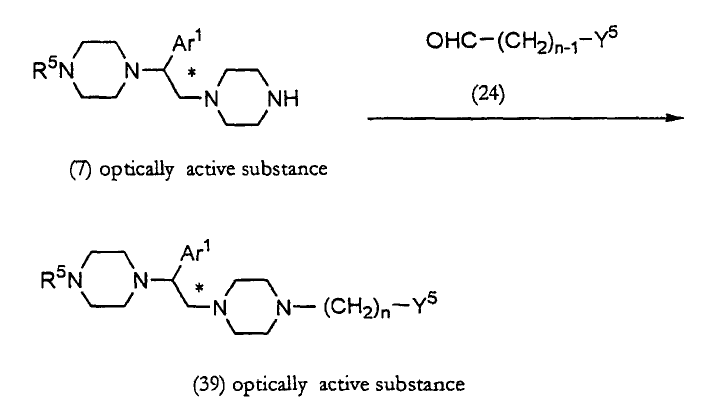

The optically active compound (39) of this invention may be obtained from the optically active compound (7) that can be prepared according to General Preparation Process 10 in the same manner as in the steps of General Preparation Process 4.

### (General Preparation Process 13)

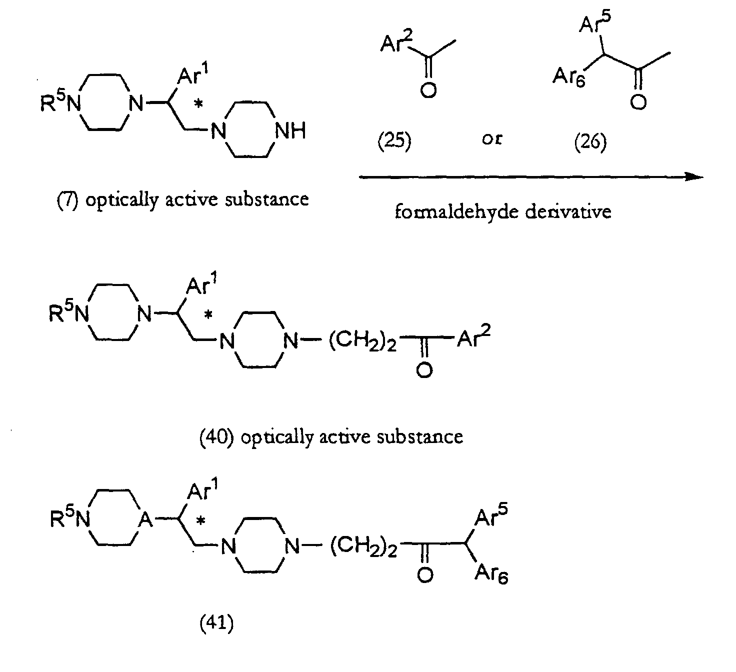

The optically active compound (40) or (41) of this invention may be obtained from the optically active compound (7) that can be prepared according to General Preparation Process 10 in the same manner as in the steps of General Preparation Process 4.

### (General Preparation Process 14)

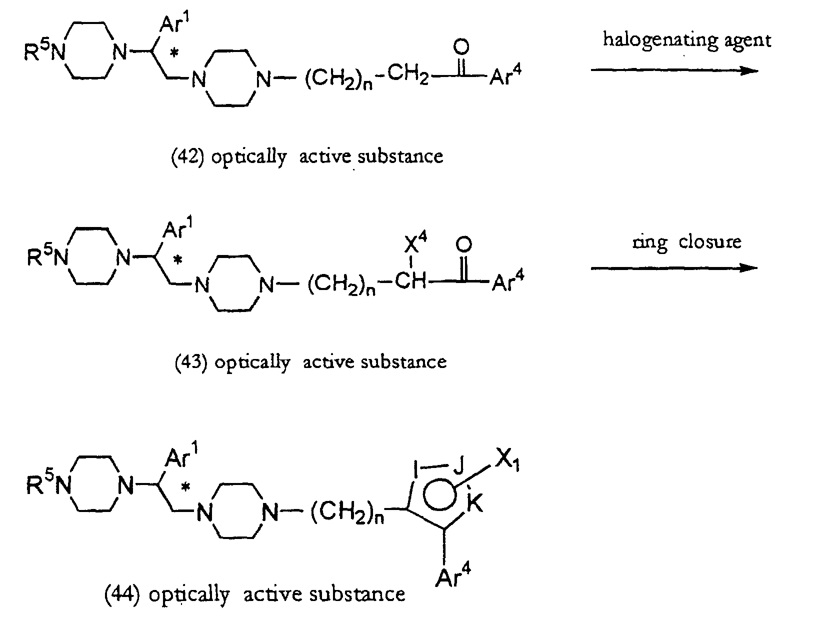

The optically active compound (44) of this invention may be obtained from the optically active compound (42) that can be prepared according to General Preparation Process 10 in the same manner as in the steps of General Preparation Process 7.

### (General Preparation Process 15)

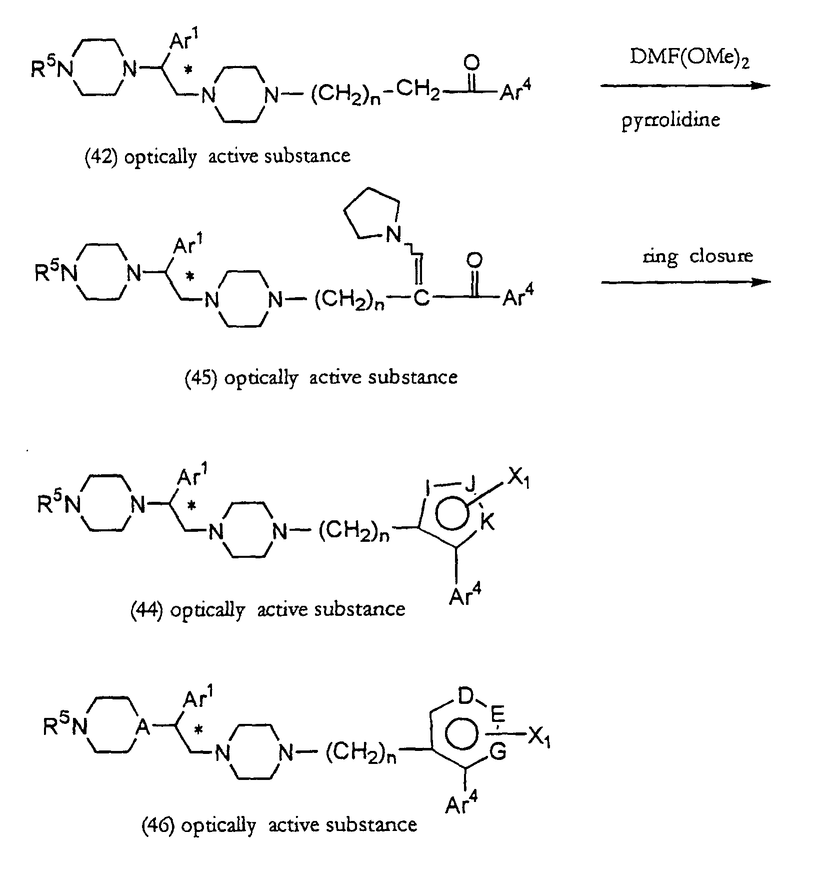

The optically active compound (44) or (46) of this invention may be obtained from the optically active compound (42) that can be prepared according to General Preparation Process 10 in the same manner as in the steps of General Preparation Process 8.

The compounds of this invention may be administered orally or parenterally. Dosage forms for administration may include tablets, capsules, granules, powders, fine powders, troches, ointments, creams, emulsions, suspensions, suppositories, injections, etc. and all the dosage forms may be prepared according to conventional formulation techniques (for example, the methods as prescribed in the Japanese Pharmacopoeia 14th Ed.). These dosage forms may be suitably selected depending on the symptom and the age of a patient as well as on therapeutic purposes. In preparing pharmaceutical preparations in various dosage forms, there may be used usual excipients (for example, crystalline cellulose, starch, lactose, mannitol, etc.), binders (for example, hydroxypropylcellulose, polyvinylpyrrolidone, etc.), lubricants (for example, magnesium stearate, talc, etc.), disintegrating agents (for example, carboxymethylcellulose calcium etc.) and others.

The dose for a compound of this invention may be 1-2000 mg/day in treatment of an adult, and it may be given once or in several divided forms daily. The dose may be suitably increased or decreased depending on the age, the body weight and the symptom of the patient.

### Best Mode for Carrying out the Invention

This invention will be more fully described by way of the following examples, but the invention is not to be limited to these examples.

### Example 1

### Synthesis of 1-[2-(4-fluorophenyl)-2-(4-isopropylpiperazino)ethyl]-4-(3-biphenyl-2-yl-propyl)piperazine tetrahydrochloride (Compound 1 in Table 1)

(1) 2-Chloro-4'-fluoroacetophenone (8.6 g) and 1-ethoxycarbonylpiperazine (16.0 g) in 60 ml of chloroform was heated under reflux for 3 hours. After cooling the reaction solution to room temperature, it was concentrated under reduced pressure, to which conc. aqueous ammonia was added; and it was extracted with ether. The organic layer was washed with a saturated aqueous sodium chloride solution and dried over anhydrous sodium sulfate. After filtering off the drying agent, the filtrate was concentrated under reduced pressure to give crude 1-ethoxycarbonyl-4-[2-(4-fluorophenyl)-2-oxoethyl]piperazine. To the crude 1-ethoxycarbonyl-4-[2-(4-fluorophenyl)-2-oxoethyl]piperazine thus obtained dissolved in 80 ml of ethanol was added 2.0 g of sodium borohydride and one drop of a 5% aqueous potassium hydroxide solution dissolved in 10 ml of water. It was heated at 50 °C for 1 hour. The reaction solution was concentrated under reduced pressure, water was then added and it was extracted with ether. The organic layer was washed with a saturated aqueous sodium chloride solution and dried over anhydrous sodium sulfate. After filtering off the drying agent, the filtrate was concentrated under reduced pressure. A 4M hydrogen chloride/ethyl acetate solution was added to the residue and it was concentrated under reduced pressure. The resulting solid was washed with ether to give 18.0 g of 1-ethoxycarbonyl-4-[2-(4-fluorophenyl)-2-hydroxyethyl]piperazine hydrochloride.
(2) To 10.0 g of 1-ethoxycarbonyl-4-[2-(4-fluorophenyl)-2-hydroxyethyl]piperazine hydrochloride were added 30 ml of benzene and 3.3 ml of thionyl chloride. It was stirred at 50 °C for 10 minutes. The reaction solution was concentrated under reduced pressure, to which 25% aqueous ammonia and water were added; and it was extracted with ethyl acetate. The organic layer was washed with a saturated aqueous sodium chloride solution and dried over anhydrous sodium sulfate. After filtering off the drying agent, the filtrate was concentrated under reduced pressure to give 10.0 g of 1-ethoxycarbonyl-4-[2-chloro-2-(4-fluorophenyl)ethyl]piperazine.
(3) To 10.0 g of 1-ethoxycarbonyl-4-[2-chloro-2-(4-fluorophenyl)ethyl]piperazine dissolved in 50 ml of benzene were added 12.1 g of 1-isopropylpiperazine dihydrochloride and 21.3 ml of diisopropylethylamine. It was stirred at 65 °C for 6 hours. To the reaction solution was added a saturated aqueous sodium bicarbonate solution, and it was extracted with ether. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate =4:1) to give 9.6 g of oily 1-ethoxycarbonyl-4-[2-(4-fluorophenyl)-2-(4-isopropylpiperazino)ethyl]piperazine.
(4) To 6.2 g of 1-ethoxycarbonyl-4-[2-(4-fluorophenyl)-2-(4-isopropylpiperazino)ethyl]piperazine dissolved in 15 ml of ethanol was added 6.2 g of potassium hydroxide. It was heated under reflux for 1 hour. After cooling the reaction solution to room temperature, water was added thereto; and it was extracted with ethyl acetate. The organic layer was washed with a saturated aqueous sodium chloride solution and dried over anhydrous sodium sulfate. After filtering off the drying agent, the filtrate was concentrated under reduced pressure to give 5.7 g of crude 1-[2-(4-fluorophenyl)-2-(4-isopropylpiperazino)ethyl]-piperazine.
(5) To 0.30 g of 1-[2-(4-fluorophenyl)-2-(4-isopropylpiperazino)ethyl]piperazine dissolved in 5 ml of dimethylformamide were added 0.24 g of 3-biphenyl-2-yl-propionic acid, 0.21 g of 1-(3,3-dimethylaminopropyl)-3-ethylcarbodimide hydrochloride, 0.18 g of 1-hydroxybenzotriazole monohydrate and 0.14 g of triethylamine. It was stirred at room temperature overnight. To the reaction solution was added a saturated aqueous sodium bicarbonate solution, and it was extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=1:1) to give 0.34 g of oily 1-[2-(4-fluorophenyl)-2-(4-isopropylpiperazino)ethyl]-4-(3-biphenyl-2-ylpropionyl)piperazine.
(6) To 0.30 g of 1-[2-(4-fluorophenyl)-2-(4-isopropylpiperazino)ethyl]-4-(3-biphenyl-2-ylpropionyl)-piperazine dissolved in 2.5 ml of tetrahydrofuran was added 21 ml of lithium aluminum hydride. It was heated under reflux for 30 minutes. After cooling the reaction solution to room temperature, 1 ml of 25% aqueous ammonia was added thereto. The precipitates thus formed were filtered off by filtration with Celite. The filtrate was concentrated under reduced pressure, and the residue was purified by silica gel column chromatography (Chromatorex NH, hexane:ethyl acetate=1:1) to give 0.19 g of oily 1-[2-(4-fluorophenyl)-2-(4-isopropylpiperazino)ethyl]-4-(3-biphenyl-2-ylpropyl)-piperazine.
(7) To 0.19 g of 1-[2-(4-fluorophenyl)-2-(4-isopropylpiperazino)ethyl]-4-(3-biphenyl-2-ylpropyl)-piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 0.20 g of 1-[2-(4-fluorophenyl)-2-(4-isopropylpiperazino)ethyl]-4-(3-biphenyl-2-ylpropyl)-piperazine tetrahydrochloride.

Structures and physical property data for this compound and the compounds obtained in like manner are shown in Table 1.

### Example 2

### Synthesis of 1-[2-(4-fluorophenyl)-2-(4-isopropylpiperazino)ethyl]-4-(3-biphenyl-2-ylallyl)piperazine tetrahydrochloride (Compound 39 in Table 1)

(1) To 0.33 g of 1-(2-(4-fluorophenyl)-2-(4-isopropylpiperazino)ethyl]piperazine obtained in Example 1-(4) dissolved in 5 ml of dimethylformamide were added 0.33 g of 2-(3-bromopropenyl)biphenyl and 0.19 g of diisopropylethylamine. It was stirred at room temperature for 2 hours. To the reaction solution was added a saturated aqueous sodium bicarbonate solution, and it was extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=1:1) to give 0.15 g of oily 1-[2-(4-fluorophenyl)-2-(4-isopropylpiperazino)ethyl]-4-(3-biphenyl-2-ylallyl)piperazine.
(2) To 0.15 g of 1-[2-(4-fluorophenyl)-2-(4-isopropylpiperazino)ethyl]-4-(3-biphenyl-2-ylallyl)piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 0.14 g of 1-[2-(4-fluorophenyl)-2-(4-isopropylpiperazino)ethyl]-4-(3-biphenyl-2-ylallyl)-piperazine tetrahydrochloride.

Structures and physical property data for this compound and the compounds obtained in like manner are shown in Table 1.

### Example 3

### Synthesis of 1-[2-(4-fluorophenyl)-2-(4-isopropylpiperazino)ethyl]-4-[3-(4'-cyanobiphenyl-2-yl)propyl]piperazine tetrahydrochloride (Compound 21 in Table 1)

(1) To 0.67 g of 1-[2-(4-fluorophenyl)-2-(4-isopropylpiperazino)ethyl]piperazine obtained in Example 1-(4) dissolved in 5 ml of methylene chloride were added 0.57 g of 2'-(3-oxopropyl)biphenyl-4-carbonitrile, 0.66 g of acetic acid and 0.51 g of sodium triacetoxyborohydride. It was stirred at room temperature for 30 minutes. To the reaction solution was added a saturated aqueous sodium bicarbonate solution, and it was extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=1:1) to give 0.75 g of oily 1-[2-(4-fluorophenyl)-2-(4-isopropylpiperazino)ethyl]-4-[3-(4'-cyanobiphenyl-2-yl)propyl)piperazine.
(2) To 0.75 g of 1-[2-(4-fluorophenyl)-2-(4-isopropylpiperazino)ethyl]-4-[3-(4'-cyanobiphenyl-2-yl)propyl]piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 0.74 g of 1-[2-(4-fluorophenyl)-2-(4-isopropylpiperazino)ethyl]-4-[3-(4'-cyanobiphenyl-2-yl)propyl]piperazine tetrahydrochloride.

Structures and physical property data for this compound and the compounds obtained in like manner are shown in Table 1.

### Example 4

### Synthesis of 1-[2-(4-fluorophenyl)-2-(4-isopropylpiperazino)ethyl]-4-[3-(4'-carbamoylbiphenyl-2-yl)propyl]-piperazine tetrahydrochloride (Compound 22 in Table 1)

(1) To 0.15 g of 1-[2-(4-fluorophenyl)-2-(4-isopropylpiperazino)ethyl]-4-[3-(4'-cyanobiphenyl-2-yl)-propyl]piperazine dissolved in 1.5 ml of *tert*-butanol was added 50 mg of potassium hydroxide. It was heated under reflux for 30 minutes. After cooling the reaction solution to room temperature, a saturated aqueous sodium bicarbonate solution was added thereto; and it was extracted with chloroform. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=1:1) to give 0.11 g of oily 1-[2-(4-fluorophenyl)-2-(4-isopropylpiperazino)ethyl]-4-[3-(4'-carbamoylbiphenyl-2-yl)propyl]piperazine.
(2) To 0.10 g of 1-[2-(4-fluorophenyl)-2-(4-isopropylpiperazino)ethyl]-4-[3-(4'-carbamoylbiphenyl-2-yl)propyl]piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 0.10 g of 1-[2-(4-fluorophenyl)-2-(4-isopropylpiperazino)ethyl]-4-[3-(4'-carbamoylbiphenyl-2-yl)propyl]piperazine tetrahydrochloride.

Structures and physical property data for this compound and the compounds obtained in like manner are shown in Table 1.

### Example 5

### Synthesis of 1-[2-(4-fluorophenyl)-2-(4-isopropylpiperazino)ethyl]-4-[3-(2-carbamoyl-4-phenylthiazol-5-yl)propyl]piperazine tetrahydrochloride (Compound 85 in Table 1)

(1) 1-[2-(4-fluorophenyl)-2-(4-isopropylpiperazino)ethyl]-4-[3-(2-ethoxycarbonyl-4-phenylthiazol-5-yl)propyl]piperazine (0.15 g) obtained in the same manner as in Example 2 was dissolved in 5 ml of a saturated ammonia/ethanol solution and it was allowed to stand overnight. The reaction solution was concentrated under reduced pressure. The residue was purified by silica gel column chromatography (Chromatorex NH, hexane:ethyl acetate=4:1) to give 0.15 g of oily 1-[2-(4-fluorophenyl)-2-(4-isopropylpiperazino)ethyl]-4-[3-(2-carbamoyl-4-phenylthiazol-5-yl)propyl]piperazine.
(2) To 0.13 g of 1-[2-(4-fluorophenyl)-2-(4-isopropylpiperazino)ethyl]-4-[3-(2-carbamoyl-4-phenylthiazol-5-yl)propyl]piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 0.15 g of 1-[2-(4-fluorophenyl)-2-(4-isopropylpiperazino)ethyl]-4-[3-(2-carbamoyl-4-phenylthiazol-5-yl)propyl]piperazine tetrahydrochloride.

Structures and physical property data for this compound and the compounds obtained in like manner are shown in Table 1.

### Example 6

### Synthesis of 1-[2-(4-fluorophenyl)-2-(4-isopropylpiperazino)ethyl]-4-[3-(4-phenylthiazol-5-yl)propyl]piperazine tetrahydrochloride (Compound 81 in Table 1)

(1) To 0.14 g of 1-[2-(4-fluorophenyl)-2-(4-isopropylpiperazino)ethyl]-4-[3-(2-ethoxycarbonyl-4-phenylthiazol-5-yl)propyl]piperazine obtained in the same manner as in Example 2 dissolved in 5 ml of ethanol was added 0.28 ml of a 1M aqueous sodium hydroxide solution. It was heated under reflux for 5 hours. After cooling the reaction solution to room temperature, 3 ml of a 4M hydrogen chloride/1,4-dioxane solution was added thereto; and it was stirred overnight. The reaction solution was concentrated under reduced pressure. A 1M aqueous sodium hydroxide solution was added to the residue, and then, it was extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=4:1) to give 90 mg of oily 1-[2-(4-fluorophenyl)-2-(4-isopropylpiperazino)ethyl]-4-[3-(4-phenylthiazol-5-yl)propyl]piperazine.
(2) To 90 mg of 1-[2-(4-fluorophenyl)-2-(4-isopropylpiperazino)ethyl]-4-[3-(4-phenylthiazol-5-yl-)propyl]piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 95 mg of 1-[2-(4-fluorophenyl)-2-(4-isopropylpiperazino)ethyl]-4-[3-(4-phenylthiazol-5-yl)propyl]piperazine tetrahydrochloride.

Structures and physical property data for this compound and the compounds obtained in like manner are shown in Table 1.

### Example 7

### Synthesis of 1-[2-(4-fluorophenyl)-2-(4-isopropylpiperazino)ethyl]-4-[3-(2-hydroxymethyl-4-phenylthiazol-5-yl)propyl]piperazine tetrahydrochloride (Compound 83 in Table 1)

(1) To 0.15 g of 1-[2-(4-fluorophenyl)-2-(4-isopropylpiperazino)ethyl]-4-[3-(2-ethoxycarbonyl-4-phenylthiazol-5-yl)propyl]piperazine obtained in the same manner as in Example 2 dissolved in 3 ml of ethanol was added 21 mg of sodium borohydride. It was stirred overnight. To the reaction solution was added a saturated aqueous sodium bicarbonate solution, and it was extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=4:1) to give 0.11 g of oily 1-[2-(4-fluorophenyl)-2-(4-isopropylpiperazino)ethyl]-4-[3-(2-hydroxymethyl-4-phenylthiazol-5-yl)propyl]piperazine.
(2) To 0.10 g of 1-[2-(4-fluorophenyl)-2-(4-isopropylpiperazino)ethyl]-4-[3-(2-hydroxymethyl-4-phenylthiazol-5-yl)propyl]piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 0.11 g of 1-[2-(4-fluorophenyl)-2-(4-isopropyl-piperazino)ethyl]-4-[3-(2-hydroxymethyl-4-phenylthiazol-5-yl)propyl]piperazine tetrahydrochloride.

Structures and physical property data for this compound and the compounds obtained in like manner are shown in Table 1.

### Example 8

### Synthesis of 1-[2-(4-fluorophenyl)-2-(4-isopropylpiperazino)ethyl]-4-[3-(2-amino-4-phenylpyrimidin-5-yl)propyl]piperazine pentahydrochloride (Compound 94 in Table 1)

(1) To 2.50 g of 1-[2-(4-fluorophenyl)-2-(4isopropylpiperazino)ethyl]-4-(5-oxo-5-phenylpentyl)piperazine obtained in the same manner as in Example 2 dissolved in 7 ml of dimethylformamide were added 7 ml of N,N-dimethylformamide dimethylacetal and 7 ml of pyrrolidine. It was heated under reflux for 3 hours. After cooling the reaction solution to room temperature, a saturated aqueous sodium bicarbonate solution was added thereto; and it was extracted with chloroform. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=4:1) to give 2.95 g of oily 1-[2-(4-fluorophenyl)-2-(4-isopropylpiperazino)ethyl]-4-(4-benzoyl-5-pyrrolidin-1-ylpent-4-enyl)piperazine.
(2) 1-[2-(4-Fluorophenyl)-2-(4-isopropylpiperazino)ethyl]-4-(4-benzoyl-5-pyrrolidin-1-ylpent-4-enyl)piperazine (0.50 g) was dissolved in 5 ml of ethanol. To this solution was added 91 mg of guanidine hydrochloride and 54 mg of potassium hydroxide in ethanol while filtering. The reaction solution was stirred at room temperature for 3 days and then heated under reflux for 9 hours. After cooling the reaction solution to room temperature, a saturated aqueous sodium bicarbonate solution was added thereto; and it was extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate =3:1) to give 0.20 g of oily 1-[2-(4-fluorophenyl)-2-(4-isopropylpiperazino)ethyl]-4-[3-(2-amino-4-phenylpyrimidin-5-yl)propyl]piperazine.
(3) To 0.20 g of 1-[2-(4-fluorophenyl)-2-(4-isopropylpiperazino)ethyl]-4-[3-(2-amino-4-phenylpyrimidin-5-yl)propyl]piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 0.19 g of 1-[2-(4-fluorophenyl)-2-(4-isopropylpiperazino)ethyl]-4-[3-(2-amino-4-phenylpyrimidin-5-yl)-propyl]piperazine pentahydrochloride.

Structures and physical property data for this compound and the compounds obtained in like manner are shown in Table 1.

### Example 9

### Synthesis of 1-[2-(4-fluorophenyl)-2-(4-isopropylpiperazino)ethyl]-4-[3-(4-phenylpyrimidin-5-yl)propyl]piperazine pentahydrochloride (Compound 93 in Table 1)

(1) To 0.50 g of 1-[2-(4-fluorophenyl)-2-(4-isopropylpiperazino)ethyl]-4-(4-benzoyl-5-pyrrolidin-1-ylpent-4-enyl)piperazine obtained in Example 8-(1), 0.55 g of ammonium formate, 5 ml of formamide and 0.1 ml of water were added. It was stirred at 180 °C for 2 hours. After cooling the reaction solution to room temperature, a saturated aqueous sodium bicarbonate solution was added thereto; and it was extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=4:1) to give 0.16 g of oily 1-[2-(4-fluorophenyl)-2-(4-isopropylpiperazino)ethyl]-4-[3-(4-phenylpyrimidin-5-yl)-propyl]piperazine.
(2) To 0.16 g of 1-[2-(4-fluorophenyl)-2-(4-isopropylpiperazino)ethyl]-4-[3-(4-phenylpyrimidin-5-yl)-propyl]piperazine dissolved in 4 ml of ethanol was added 1 ml of a 4M hydrogen chloride/1,4-dioxane solution. The reaction solution was concentrated under reduced pressure and then the residue was crystallized from ethyl acetate/methanol. Crystals were recovered by filtration and washed with ethyl acetate to give 0.15 g of 1-[2-(4-fluorophenyl)-2-(4-isopropylpiperazino)ethyl]-4-[3-(4-phenylpyrimidin-5-yl)propyl]piperazine pentahydrochloride.

Structures and physical property data for this compound and the compounds obtained in like manner are shown in Table 1.

### Example 10

### Synthesis of 1-[2-(4-fluorophenyl)-2-(4-isopropylpiperazino)ethyl]-4-[3-(2-amino-4-phenylthiazol-5-yl)propyl]piperazine tetrahydrochloride (Compound 84 in Table 1)

(1) 1-[2-(4-Fluorophenyl)-2-(4-isopropylpiperazino)ethyl]-4-(5-oxo-5-phenylpentyl)piperazine (0.20 g) obtained in the same manner as in Example 2 was dissolved in 8 ml of a mixed solvent of chloroform and acetic acid (2:1). To this solution was added dropwise 47 mg of bromine dissolved in 2 ml of a mixed solvent of chloroform and acetic acid (2:1) over 10 minutes. The reaction solution was concentrated under reduced pressure to give 0.22 g of oily 1-[2-(4-fluorophenyl)-2-(4-isopropylpiperazino)ethyl]-4-(4-bromo-5-oxo-5-phenylpentyl)piperazine.
(2) To 0.20 g of 1-[2-(4-fluorophenyl)-2-(4-isopropylpiperazino)ethyl]-4-(4-bromo-5-oxo-5-phenylpentyl)piperazine dissolved in 2 ml of ethanol was added 19 mg of thiourea. It was heated under reflux for 1 hour. After cooling the reaction solution to room temperature, a saturated aqueous sodium bicarbonate solution was added thereto; and it was extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=3:1) to give 70 mg of oily 1-[2-(4-fluorophenyl)-2-(4-isopropylpiperazino)ethyl]-4-[3-(2-amino-4-phenylthiazol-5-yl)propyl]piperazine.
(3) To 70 mg of 1-[2-(4-fluorophenyl)-2-(4-isopropylpiperazino)ethyl]-4-[3-(2-amino-4-phenylthiazol-5-yl)propyl]piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 70 mg of 1-[2-(4-fluorophenyl)-2-(4-isopropylpiperazino)ethyl]-4-[3-(2-amino-4-phenylthiazol-5-yl)propyl]piperazine tetrahydrochloride.

Structures and physical property data for this compound and the compounds obtained in like manner are shown in Table 1.

### Example 11

### Synthesis of 1-[2-(4-fluorophenyl)-2-(4-isopropylpiperazino)ethyl]-4-[3-(3-hydroxy-5-phenyl-1H-pyrazol-4-yl)propyl]piperazine tetrahydrochloride (Compound 92 in Table 1)

(1) To 0.13 g of 1-[2-(4-fluorophenyl)-2-(4-isopropylpiperazino)ethyl]-4-(4-ethoxycarbonyl-5-oxo-5-phenylpentyl)piperazine obtained in the same manner as in Example 2 dissolved in 2 ml of ethanol was added 0.12 g of hydrazine hydrate. It was heated under reflux for 3 hours. After cooling the reaction solution to room temperature, a saturated aqueous sodium bicarbonate solution was added thereto; and it was extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=3:1) to give 0.11 g of oily 1-[2-(4-fluorophenyl)-2-(4-isopropylpiperazino)ethyl]-4-[3-(3-hydroxy-5-phenyl-1H-pyrazol-4-yl)propyl]piperazine.
(2) To 0.11 g of 1-[2-(4-fluorophenyl)-2-(4-isopropylpiperazino)ethyl)-4-[3-(3-hydroxy-5-phenyl-1H-pyrazol-4-yl)propyl]piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 0.10 g of 1-[2-(4-fluorophenyl)-2-(4-isopropylpiperazino)ethyl]-4-[3-(3-hydroxy-5-phenyl-1H-pyrazol-4-yl)propyl]piperazine tetrahydrochloride.

Structures and physical property data for this compound and the compounds obtained in like manner are shown in Table 1.

### Example 12

### Synthesis of 1-[2-(4-fluorophenyl)-2-(4-isopropylpiperazino)ethyl]-4-[3-[2-(2-aminothiazol-4-yl)phenyl]propyl]piperazine tetrahydrochloride (Compound 60 in Table 1)

(1) To 0.55 g of 1-[2-(4-fluorophenyl)-2-(4-isopropylpiperazino)ethyl]-4-[3-(2-acetylphenyl)propionyl]piperazine obtained in the same manner as in the steps up to Example 1-(5) were added 1.3 ml of a 25% hydrobromide/acetic acid solution and 5 ml of chloroform. It was stirred for 10 minutes. To the solution was added dropwise a chloroform solution (2 ml) of 0.21 g of bromine. The reaction solution was stirred at room temperature for 1 hour and then concentrated under reduced pressure. The residue was dissolved in 10 ml of ethanol, to which 0.25 g of thiourea was added; and it was heated under reflux for 30 minutes. After cooling the reaction solution to room temperature, a saturated aqueous sodium bicarbonate solution was added thereto; and it was extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate =1:1) to give 0.44 g of oily 1-[2-(4-fluorophenyl)-2-(4-isopropylpiperazino)ethyl]-4-[3-[2-(2-aminothiazol-4-yl)phenyl]propionyl]piperazine.
(2) To 0.34 g of 1-[2-(4-fluorophenyl)-2-(4-isopropylpiperazino)ethyl]-4-[3-[2-(2-aminothiazol-4-yl)phenyl]propionyl]piperazine dissolved in 5 ml of tetrahydrofuran was added 23 mg of lithium aluminum hydride. It was heated under reflux for 1 hour. After cooling the reaction solution to room temperature, 1 ml of 25% aqueous ammonia was added thereto. The resulting precipitates were filtered off by filtration with Celite. The filtrate was concentrated under reduced pressure. The residue was purified by silica gel column chromatography (Chromatorex NH, hexane:ethyl acetate=4:1) to give 0.22 g of oily 1-[2-(4-fluorophenyl)-2-(4-isopropylpiperazino)ethyl]-4-[3-[2-(2-aminothiazol-4-yl)phenyl]propyl]piperazine.
(3) To 0.19 g of 1-(2-(4-fluorophenyl)-2-(4-isopropylpiperazino)ethyl]-4-[3-[2-(2-aminothiazol-4-yl)phenyl]propyl]piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 0.20 g of 1-[2-(4-fluorophenyl)-2-(4-isopropylpiperazino)ethyl]-4-[3-[2-(2-aminothiazol-4-yl)phenyl]propyl]piperazine tetrahydrochloride.

Structures and physical property data for this compound and the compounds obtained in like manner are shown in Table 1.

### Example 13

### Synthesis of 1-[2-(4-fluorophenyl)-2-(4-isopropyl-piperazino)ethyl]-4-[3-(4'-fluorobiphenyl-2-yl)-3-oxopropyl]piperazine tetrahydrochloride (Compound 46 in Table 1)

(1) To 5.7 g of 1-[2-(4-fluorophenyl)-2-(4-isopropylpiperazino)ethyl]piperazine obtained in Example 1-(4) dissolved in 40 ml of ethanol was added 20 ml of a 4M hydrogen chloride/1,4-dioxane solution. The reaction solution was concentrated under reduced pressure, and then, the resulting solid was then washed with ethyl acetate to give 5.3 g of 1-[2-(4-fluorophenyl)-2-(4-isopropylpiperazino)ethyl]piperazine tetrahydrochloride.
(2) To 0.50 g of 1-[2-(4-fluorophenyl)-2-(4-isopropylpiperazino)ethyl]piperazine tetrahydrochloride were added 0.27 g of 2-acetyl-4'-fluorobiphenyl, 0.1 ml of conc. hydrochloric acid, 3 ml of 1,3-dioxolan and 5 ml of diethyleneglycol. It was stirred at 150 °C for 30 minutes. After cooling the reaction solution to room temperature, toluene was added thereto. It was washed with a saturated aqueous sodium bicarbonate solution and a saturated aqueous sodium chloride solution. The organic layer was dried over anhydrous sodium sulfate. After filtering off the drying agent, the filtrate was concentrated under reduced pressure. The residue was purified by silica gel column chromatography (Chromatorex NH, hexane:ethyl acetate=10:1, and Wakogel C200, chloroform:methanol=20:1) to give 80 mg of oily 1-[2-(4-fluorophenyl)-2-(4-isopropylpiperazino)ethyl]-4-[3-(4'-fluorobiphenyl-2-yl)-3-oxopropyl]piperazine.
(3) To 80 mg of 1-[2-(4-fluorophenyl)-2-(4-isopropylpiperazino)ethyl]-4-[3-(4'-fluorobiphenyl-2-yl)-3-oxo-propyl]piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 65 mg of 1-[2-(4-fluorophenyl)-2-(4-isopropylpiperazino)ethyl]-4-[3-(4'-fluorobiphenyl-2-yl)-3-oxo-propyl]piperazine tetrahydrochloride.

### Example 14

### Synthesis of 1-[2-(4-fluorophenyl)-2-(1-isopropylpiperazin-4-yl)ethyl]-4-[3-biphenyl-2-ylpropyl)piperazine trihydrochloride (Compound 29 in Table 1)

(1) To 48.3 ml of diisopropylamine dissolved in 200 ml of tetrahydrofuran was added dropwise at 0 °C 137 ml of a 2.5 M n-butyl lithium/hexane solution. To the reaction solution was added dropwise 100 ml of 25.2 g of p-fluorophenylacetic acid in tetrahydrofuran and then 28.4 ml of hexamethylphosphoric triamide (HMPA) was added. The reaction solution was allowed to raise to room temperature, and it was stirred for 30 minutes. The reaction solution was cooled to 0 °C, to which 32.5 g of 1-*tert*-butoxycarbonyl-4-piperidone in tetrahydrofuran (100 ml) was added dropwise. After allowing the reaction solution to raise to room temperature, it was stirred for 3 hours. To the reaction solution was added water; and it was extracted with ethyl acetate. The aqueous layer was made acidic by addition of potassium hydrogensulfate, and it was extracted with ethyl acetate. The organic layer was washed with a saturated aqueous sodium chloride solution and then dried over anhydrous sodium sulfate. After filtering off the drying agent, the filtrate was concentrated under reduced pressure. Ether was added to the residue; and it was stirred at room temperature. The crystals thus separated were recovered by filtration and then washed with ether to give 30.0 g of powdery 1-*tert*-butoxycarbonyl-4-[carboxy-(4-fluorophenyl)methyl]-4-hydroxypiperidine.
(2) To a suspension of 30.0 g of 1-*tert*-butoxycarbonyl- 4-[carboxy-(4-fluorophenyl)methyl]-4-hydroxypiperidine in 60 ml of chloroform was added dropwise at 0 °C 60 ml of conc. sulfuric acid. The reaction solution was heated under reflux for 3 hours and cooled to 0 °C. To the reaction solution were added 400 ml of a 4M aqueous solution of sodium hydroxide, 200 ml of 1,4-dioxane and 22.2 g of di-*tert*-butyldicarbonate. After stirring the reaction solution at room temperature for 30 minutes, it was made acidic by addition of potassium hydrogensulfate and extracted with chloroform. The organic layer was washed with a saturated aqueous sodium chloride solution and dried over anhydrous sodium sulfate. After filtering off the drying agent, the filtrate was concentrated under reduced pressure. The residue was purified by silica gel column chromatography (WAKOGEL C200, chloroform:methanol=10:1) to give 28.3 g of oily 1-*tert*-butoxycarbonyl-4-[carboxy-(4-fluorophenyl)methyl]-3,6-dihydro-2H-pyridine.
(3) To 28.3 g of 1-*tert*-butoxycarbonyl-4-[carboxy-(4-fluorophenyl)methyl]-3,6-dihydro-2H-pyridine dissolved in 300 ml of methanol was added 2.80 g of palladium hydroxide/carbon; and it was stirred at room temperature under hydrogen atmosphere for 2 days. The catalyst was filtered off by filtration with Celite. The filtrate was concentrated under reduced pressure to give 28.0 g of crude 1-*tert*-butoxycarbonyl-4-[carboxy-(4-fluorophenyl)-methyl]piperidine.
(4) To 9.2 g of 1-*tert*-butoxycarbonyl-4-[carboxy-(4-fluorophenyl)methyl]piperidine in 100 ml of dimethylformamide were added 6.6 g of 1-benzyloxycarbonylpiperazine, 6.3 g of 1-(3,3-dimethylaminopropyl)- 3-ethylcarbodiimide hydrochloride, 6.3 g of 1-hydroxybenzotriazole hydrate and 4.5 ml of triethylamine. It was stirred at room temperature for 3 hours. To the reaction solution was added a saturated aqueous sodium bicarbonate solution and extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=4:1) to give 11.0 g of oily 1-benzyloxycarbonyl-4-[2-(1-tert-butoxycarbonylpiperidin-4-yl)-2-(4-fluorophenyl)acetyl]piperazine.
(5) To 10.6 g of 1-benzyloxycarbonyl-4-[2-(1-*tert*-butoxycarbonylpiperidin-4-yl)-2-(4-fluorophenyl)acetyl]-piperazine dissolved in 100 ml of methanol was added 50 ml of a 4M hydrogen chloride/1,4-dioxane solution. It was stirred at room temperature for 1 hour. The reaction solution was concentrated under reduced pressure. Ethyl acetate was added to the residue, and it was washed with a 1M aqueous sodium hydroxide solution and a saturated aqueous sodium chloride solution. The organic layer was dried over anhydrous sodium sulfate. After filtering off the drying agent, the filtrate was concentrated under reduced pressure. The residue was dissolved in 100 ml of dimethylformamide, to which 2.95 ml of 2-iodopropane and 8.1 g of potassium carbonate were added; and it was stirred at room temperature overnight. To the reaction solution was added ethyl acetate, and it was washed with water and a saturated aqueous sodium chloride solution. The organic layer was dried over anhydrous sodium sulfate. After filtering off the drying agent, the filtrate was concentrated under reduced pressure. The residue was purified by silica gel column chromatography (Chromatorex NH, hexane:ethyl acetate=4:1) to give 6.2 g of oily 1-benzyloxycarbonyl-4-[2-(4-fluorophenyl)-2-(1-isopropylpiperidin-4-yl)acetylpiperazine.
(6) To 6.1 g of 1-benzyloxycarbonyl-4-[2-(4-fluorophenyl)-2-(1-isopropylpiperidin-4-yl)acetylpiperazine dissolved in 60 ml of methanol was added 0.60 g of palladium hydroxide/carbon. It was stirred at room temperature under hydrogen atmosphere overnight. The catalyst was filtered off by filtration with Celite. The filtrate was concentrated under reduced pressure to give 4.80 g of oily 2-(4-fluorophenyl)-2-(1-isopropylpiperidin-4-yl)-1-piperazin-1-ylethanone.
(7) To 0.30 g of 2-(4-fluorophenyl)-2-(1-isopropylpiperidin-4-yl)-1-piperazin-1-ylethanone dissolved in 5 ml of dimethylformamide were added 0.22 g of 3-biphenyl-2-ylpropionic acid, 0.20 g of 1-(3,3-dimethylaminopropyl)-3-ethylcarbodiimide hydrochloride, 0.20 g of 1-hydroxybenzotriazole hydrate and 0.14 ml of triethylamine. It was stirred at room temperature for 3 hours. To the reaction solution was added a saturated aqueous sodium bicarbonate solution and extracted with ethyl acetate. The organic layer was washed with a saturated aqueous sodium chloride solution and dried over anhydrous sodium sulfate. After filtering off the drying agent, the filtrate was concentrated under reduced pressure. The residue was purified by silica gel column chromatography (Chromatorex NH, hexane:ethyl acetate=2:1) to give 0.30 g of oily 3-biphenyl-2-yl-1-[4-[2-(4-fluorophenyl)-2-(1-isopropylpiperidin-4-yl)acetyl]piperazin-1-yl]propane-1-onë.
(8) To 0.28 g of 3-biphenyl-2-yl-l-[4-[2-(4-fluorophenyl)-2-(1-isopropylpiperidin-4-yl)acetyl]-piperazin-1-yl]propane-1-one dissolved in 5 ml of tetrahydrofuran was added 40 mg of lithium aluminum hydride. It was heated under reflux for 30 minutes. After cooling the reaction solution to room temperature, 1 ml of 25% aqueous ammonia was added thereto. The resulting precipitates were filtered off by filtration with Celite. The filtrate was then concentrated under reduced pressure. The residue was purified by silica gel column chromatography (Chromatorex NH, hexane:ethyl acetate=4:1) to give 0.20 g of oily 1-[2-(4-fluorophenyl)-2-(1-isopropylpiperidin-4-yl)ethyl]-4-(3-biphenyl-2-ylpropyl)piperazine.
(9) To 0.18 g of 1-[2-(4-fluorophenyl)-2-(1-isopropylpiperidin-4-yl)ethyl]-4-(3-biphenyl-2-ylpropyl)-piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 0.20 g of 1-[2-(4-fluorophenyl)-2-(1-isopropylpiperidin-4-yl)ethyl]-4-(3-biphenyl-2-ylpropyl)piperazine trihydrochloride.

Structures and physical property data for this compound and the compounds obtained in like manner are shown in Table 1.

### Example 15

### Synthesis of 1-[2-(4-fluorophenyl)-2-(1-isopropylpiperidin-4-yl)ethyl]-4-(4-cyclooctyl-4-oxobutyl)piperazine trihydrochloride (Compound 101 in Table 1)

(1) To 4.1 g of 2-(4-fluorophenyl)-2-(1-isopropylpiperidin-4-yl)-1-piperazin-1-ylethanone obtained in Example 14-(6) dissolved in 40 ml of tetrahydrofuran was added 0.45 g of lithium aluminum hydride. It was heated under reflux for 30 minutes. After cooling the reaction solution to room temperature, 5 ml of 25% aqueous ammonia was added thereto. The resulting precipitates were filtered off by filtration with Celite. The filtrate was concentrated under reduced pressure. The residue was purified by silica gel column chromatography (Chromatorex NH, chloroform:methanol=50:1) to give 3.4 g of oily 1-[2-(4-fluorophenyl)-2-(1-isopropylpiperidin-4-yl)ethyl]piperazine.
(2) To 0.30 g of 1-[2-(4-fluorophenyl)-2-(1-isopropylpiperidin-4-yl)ethyl]piperazine dissolved in 3 ml of N,N-dimethylformamide were added 0.35 g of 4-bromo-1-cyclooctylbutan-1-one and 0.31 ml of diisopropylethylamine. It was stirred at 70 °C for 3 hours. After cooling the reaction solution to room temperature, ethyl acetate was added thereto. It was then washed with a saturated aqueous sodium bicarbonate solution and a saturated aqueous sodium chloride solution. The organic layer was dried over anhydrous sodium sulfate. After filtering off the drying agent, the filtrate was concentrated under reduced pressure. The residue was purified by silica gel column chromatography (Chromatorex NH, hexane:ethyl acetate=4:1) to give 0.29 g of oily 1-[2-(4-fluorophenyl)-2-(1-isopropylpiperidin-4-yl)ethyl]-4-(4-cyclooctyl-4-oxobutyl)piperazine.
(3) To 0.29 g of 1-[2-(4-fluorophenyl)-2-(1-isopropylpiperidin-4-yl)ethyl]-4-(4-cyclooctyl-4-oxobutyl)-piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 0.28 g of 1-[2-(4-fluorophenyl)-2-(1-isopropylpiperidin-4-yl)ethyl]-4-(4-cyclooctyl-4-oxobutyl)piperazine trihydrochloride.

### Example 16

### Synthesis of 1-[2-(4-fluorophenyl)-2-(1-isopropylpiperidin-4-yl)ethyl]-4-[3-(4'-fluorobiphenyl-2-yl)-3-oxopropyl]piperazine trihydrochloride (Compound 47 in Table 1)

(1) To 3.4 g of 1-[2-(4-fluorophenyl)-2-(1-isopropylpiperidin-4-yl)ethyl]piperazine obtained in Example 15-(1) dissolved in 40 ml of ethanol was added 10 ml of a 4M hydrogen chloride/1,4-dioxane solution. The reaction solution was concentrated under reduced pressure, and then, the residue was crystallized from ethyl acetate/methanol. Crystals were recovered by filtration and washed with ethyl acetate to give 3.6 g of 1-[2-(4-fluorophenyl)-2-(1-isopropylpiperidin-4-yl)ethyl]piperazine trihydrochloride.
(2) To 0.50 g of 1-[2-(4-fluorophenyl)-2-(1-isopropylpiperadin-4-yl)ethyl]piperazine tetrahydrochloride, 0.27 g of 2-acetyl-4'-fluorobiphenyl, 0.1 ml of conc. hydrochloric acid, 3 ml of 1,3-dioxolan and 5 ml of diethylene glycol were added. It was stirred at 150 °C for 30 minutes. After cooling the reaction solution to room temperature, toluene was added thereto. It was then washed with a saturated aqueous sodium bicarbonate solution and a saturated aqueous sodium chloride solution. The organic layer was dried over anhydrous sodium sulfate. After filtering off the drying agent, the filtrate was concentrated under reduced pressure. The residue was purified by silica gel column chromatography (Chromatorex NH, hexane:ethyl acetate=10:1, and WAKOGEL C200, chloroform:methanol=20:1) to give 80 mg of oily 1-[2-(4-fluorophenyl)-2-(4-isopropylpiperidin-4-yl)ethyl]-4-(3-(4'-fluorobiphenyl-2-yl)-3-oxo-propyl]piperazine.
(3) To 80 mg of 1-[2-(4-fluorophenyl)-2-(4-isopropylpiperidin-4-yl)ethyl]-4-[3-(4'-fluorobiphenyl-2-yl)-3-oxo-propyl]piperazine dissolved in 4 ml of ethanol was added 10 ml of a 4M hydrogen chloride/1,4-dioxane solution. The reaction solution was concentrated under reduced pressure, and then, the residue was crystallized from ethyl acetate/methanol. Crystals were recovered by filtration and then washed with ethyl acetate to give 75 mg of 1-[2-(4-fluorophenyl)-2-(1-isopropylpiperidin-4-yl)ethyl]-4-[3-(4'-fluorobiphenyl-2-yl)-3-oxo-propyl]piperazine trihydrochloride.

### Example 17

### Synthesis of 1-[2-(4-fluorophenyl)-2-(4-isopropylpiperazino)ethyl]-4-[4,4-bis-(4-fluorophenyl)-4-carboxybutyl]piperazine tetrahydrochloride (Compound 104 in Table 1)

(1) 1-[2-(4-Fluorophenyl)-2-(4-isopropylpiperazino)ethyl]-4-[4,4-bis-(4-fluorophenyl)-4-methoxycarboxybutyl]piperazine (0.50 g) obtained in the same manner as in Example 2 was dissloved in 20 ml of conc. hydrochloric acid. It was heated under reflux overnight. The reaction solution was concentrated under reduced pressure to give 0.44 g of 1-[2-(4-fluorophenyl)-2-(4-isopropylpiperazino)ethyl]-4-[4,4-bis-(4-fluorophenyl)-4-carboxybutyl]piperazine tetrahydrochloride.

Structures and physical property data for this compound and the compounds obtained in like manner are shown in Table 1.

### Example 18

### Synthesis of 1-[2-(4-fluorophenyl)-2-(4-isopropylpiperazino)ethyl]-4-[4,4-bis-(4-fluorophenyl)-4-carbamoylbutyl]piperazine tetrahydrochloride (Compound 105 in Table 1)

(1) To 0.20 g of 1-[2-(4-fluorophenyl)-2-(4-isopropylpiperazino)ethyl]-4-[4,4-bis-(4-fluorophenyl)-4-carboxybutyl]piperazine obtained in Example 17 dissolved in 5 ml of thionyl chloride was added 0.1 ml of dimethylformamide. It was stirred at 80 °C for 2 hours. The reaction solution was concentrated under reduced pressure. The residue was dissolved in 5 ml of tetrahydrofuran, to which 5 ml of conc. aqueous ammonia was added. It was stirred at room temperature for 2 hours. Ethyl acetate was added to the reaction solution and washed with a saturated aqueous sodium bicarbonate solution and a saturated aqueous sodium chloride solution. The organic layer was dried over anhydrous sodium sulfate. After filtering off the drying agent, the filtrate was concentrated under reduced pressure. The residue was purified by silica gel column chromatography (Chromatorex NH, hexane:ethyl acetate=1:1) to give 80 mg of oily 1-[2-(4-fluorophenyl)-2-(4-isopropylpiperazino)ethyl]-4-[4,4-bis-(4-fluorophenyl)-4-carbamoylbutyl]piperazine.
(2) To 80 mg of 1-[2-(4-fluorophenyl)-2-(4-isopropylpiperazino)ethyl]-4-[4,4-bis-(4-fluorophenyl)-4-carbamoylbutyl]piperazine dissolved in 4 ml of ethanol was added 1 ml of a 4M hydrogen chloride/1,4-dioxane solution. The reaction solution was concentrated under reduced pressure, and then, the residue was crystallized from ethyl acetate/methanol. Crystals were recovered by filtration and then washed with ethyl acetate to give 75 mg of 1-[2-(4-fluorophenyl)-2-(4-isopropylpiperazino)ethyl]-4-[4,4-bis-(4-fluorophenyl)-4-carbamoylbutyl]piperazine tetrahydrochloride.

Structures and physical property data for this compound and the compounds obtained in like manner are shown in Table 1.

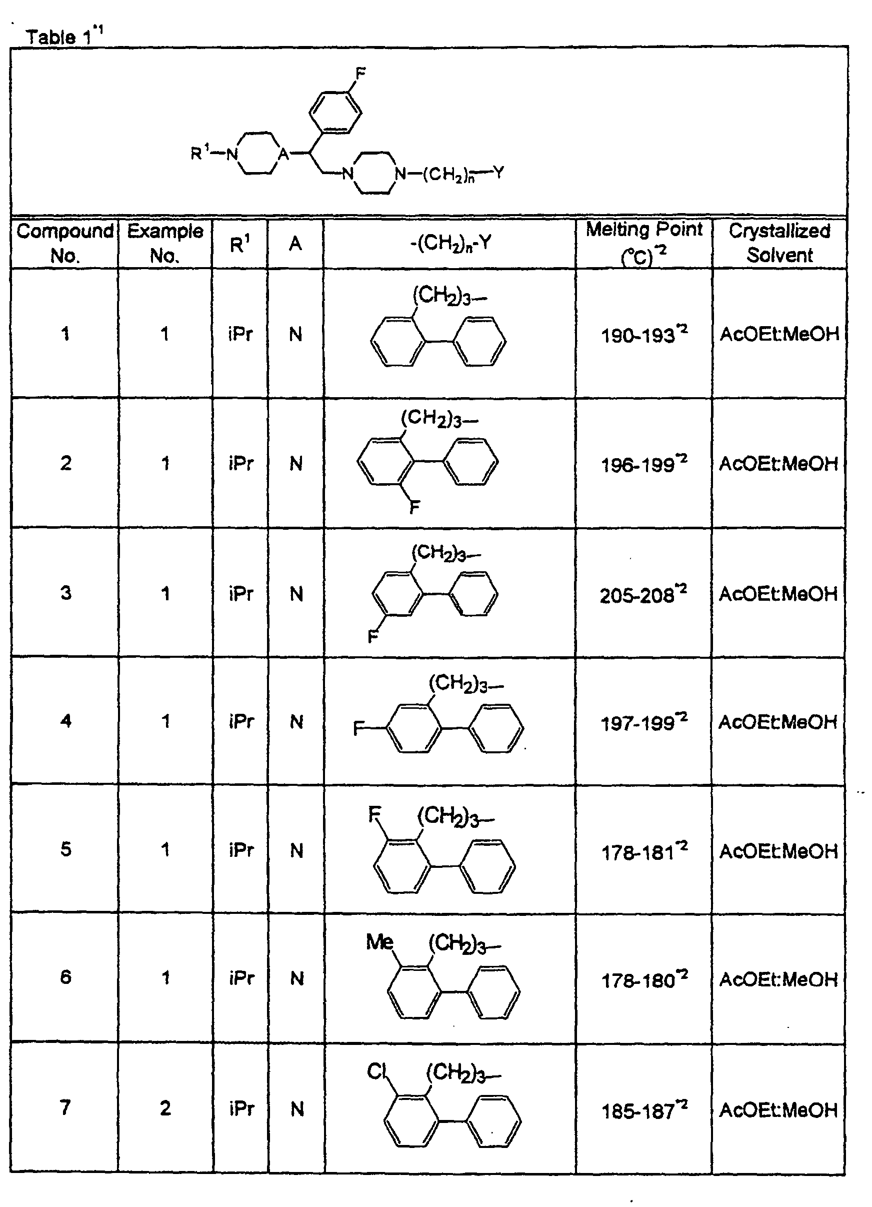

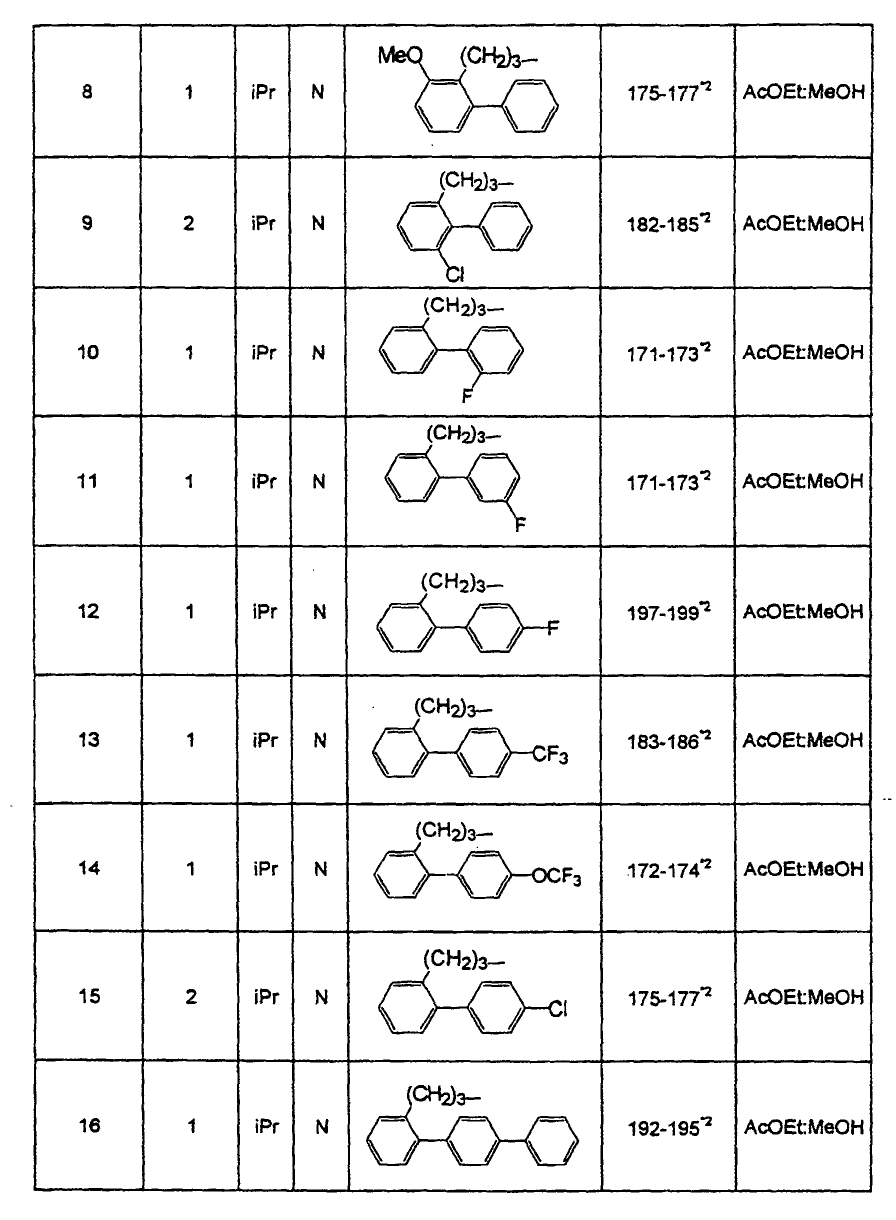

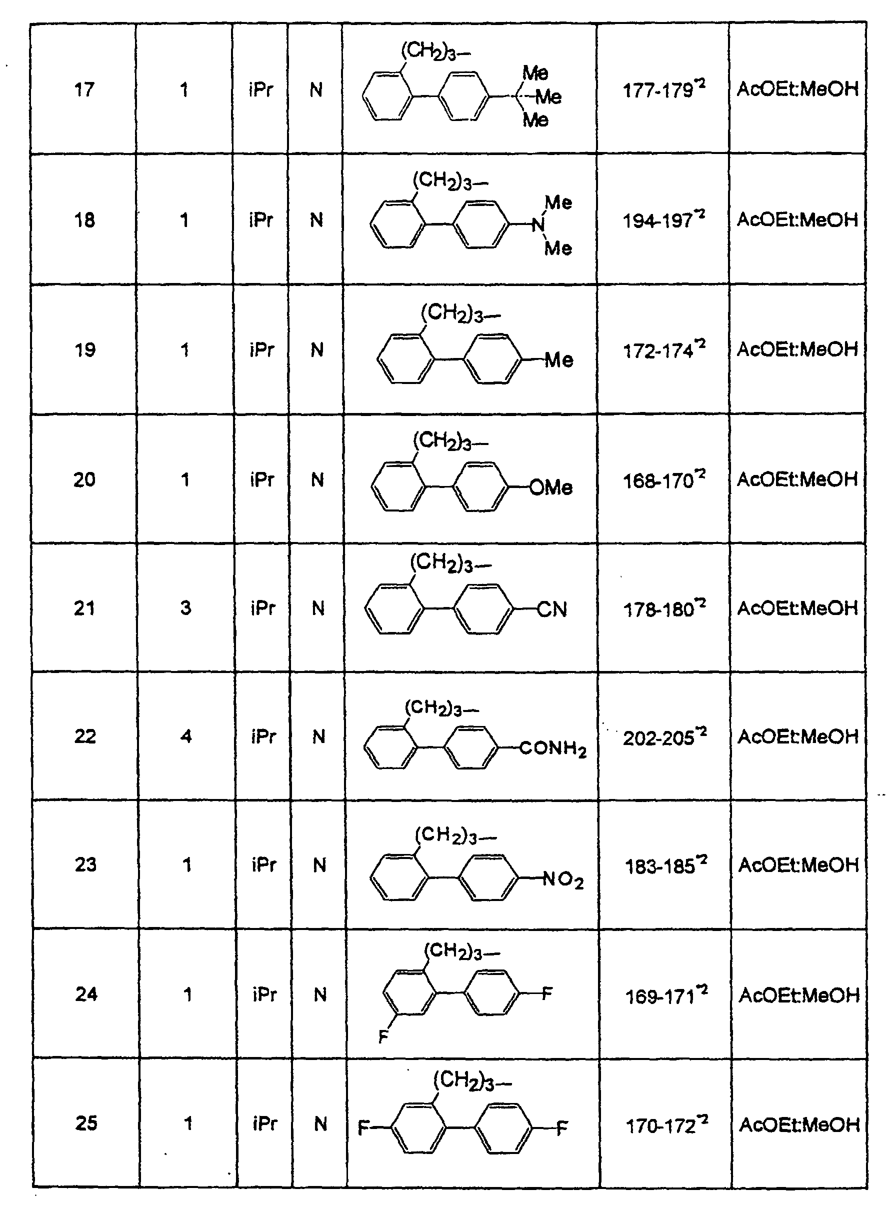

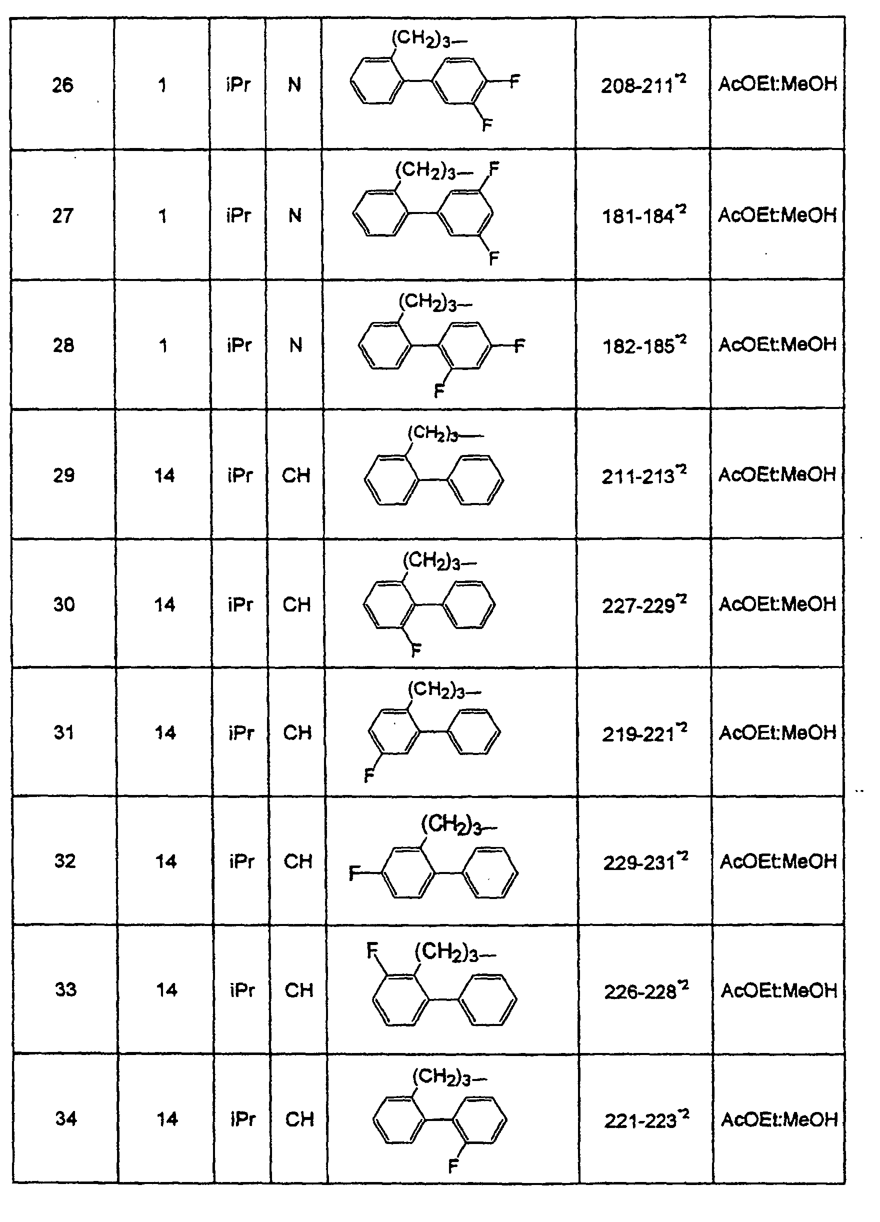

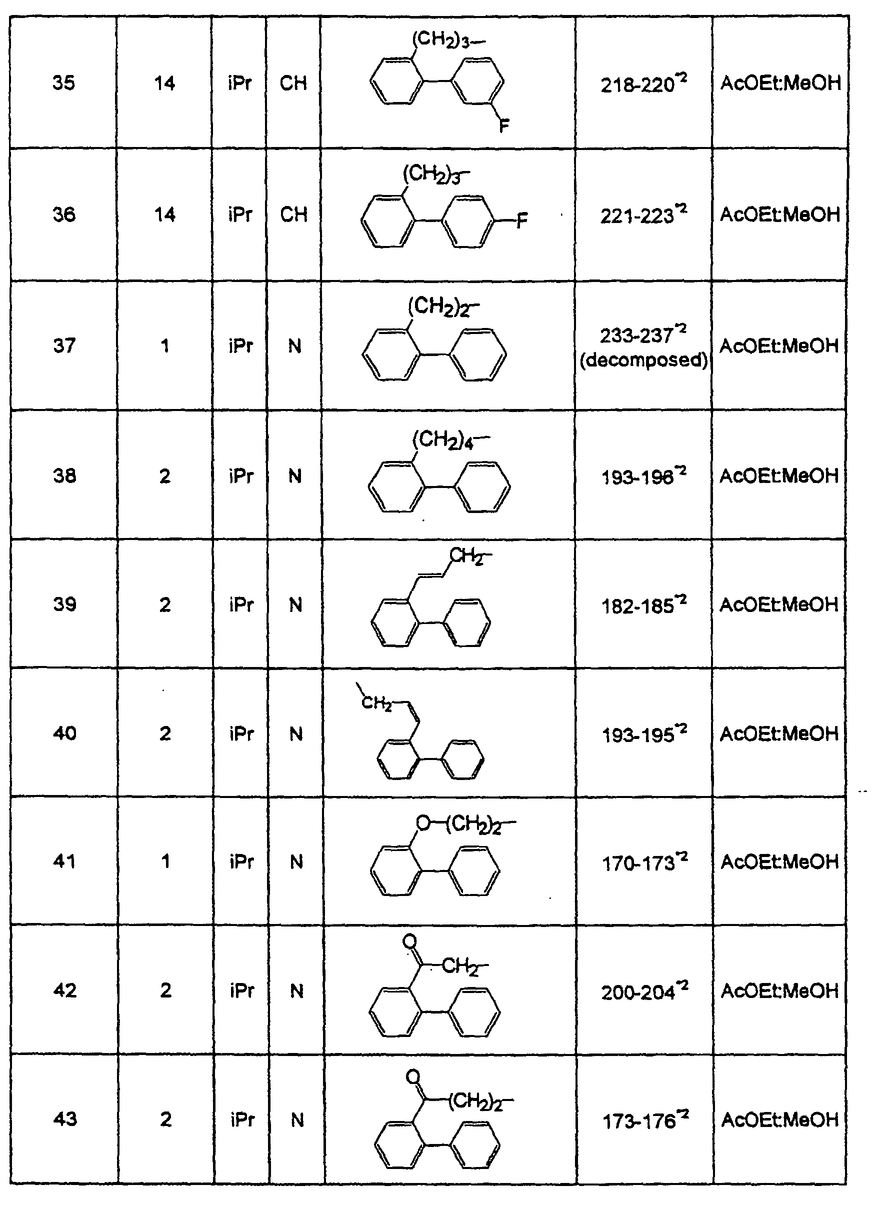

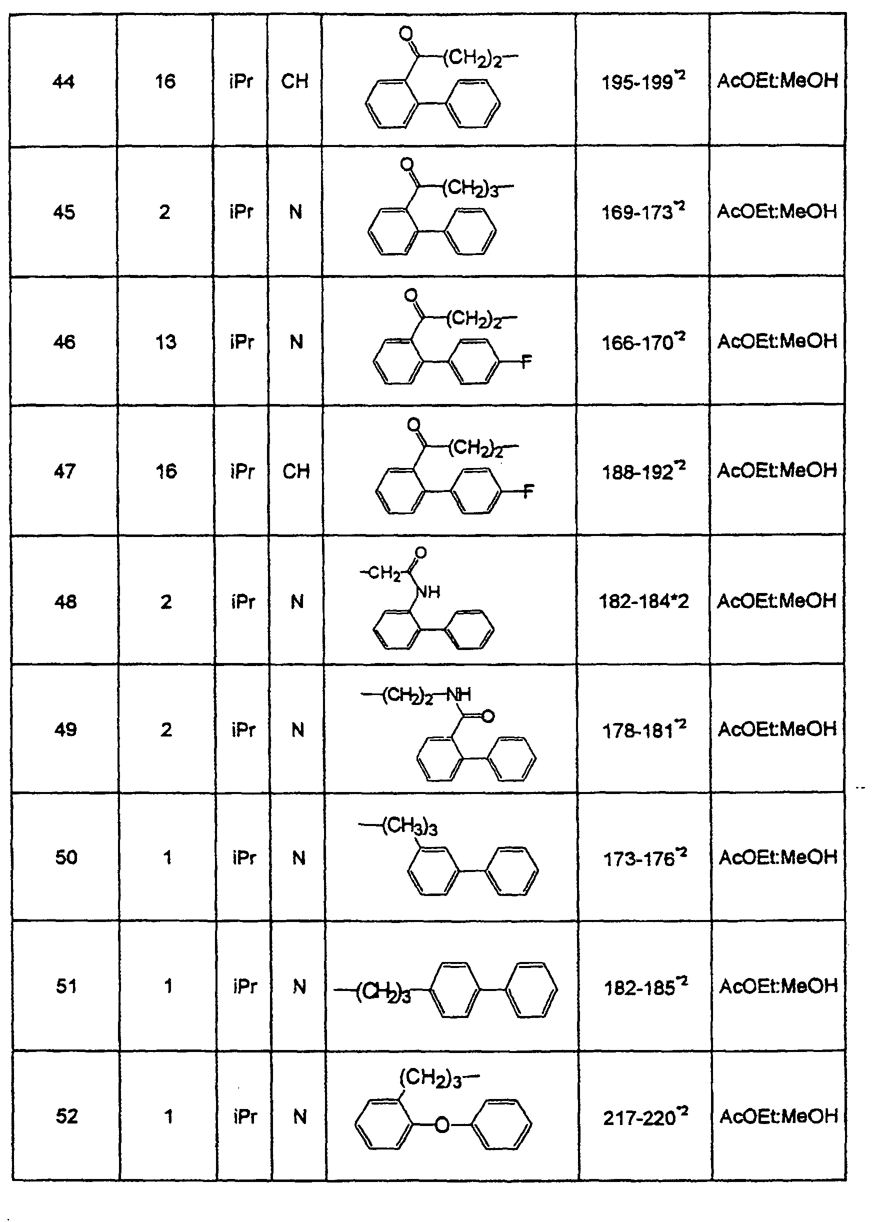

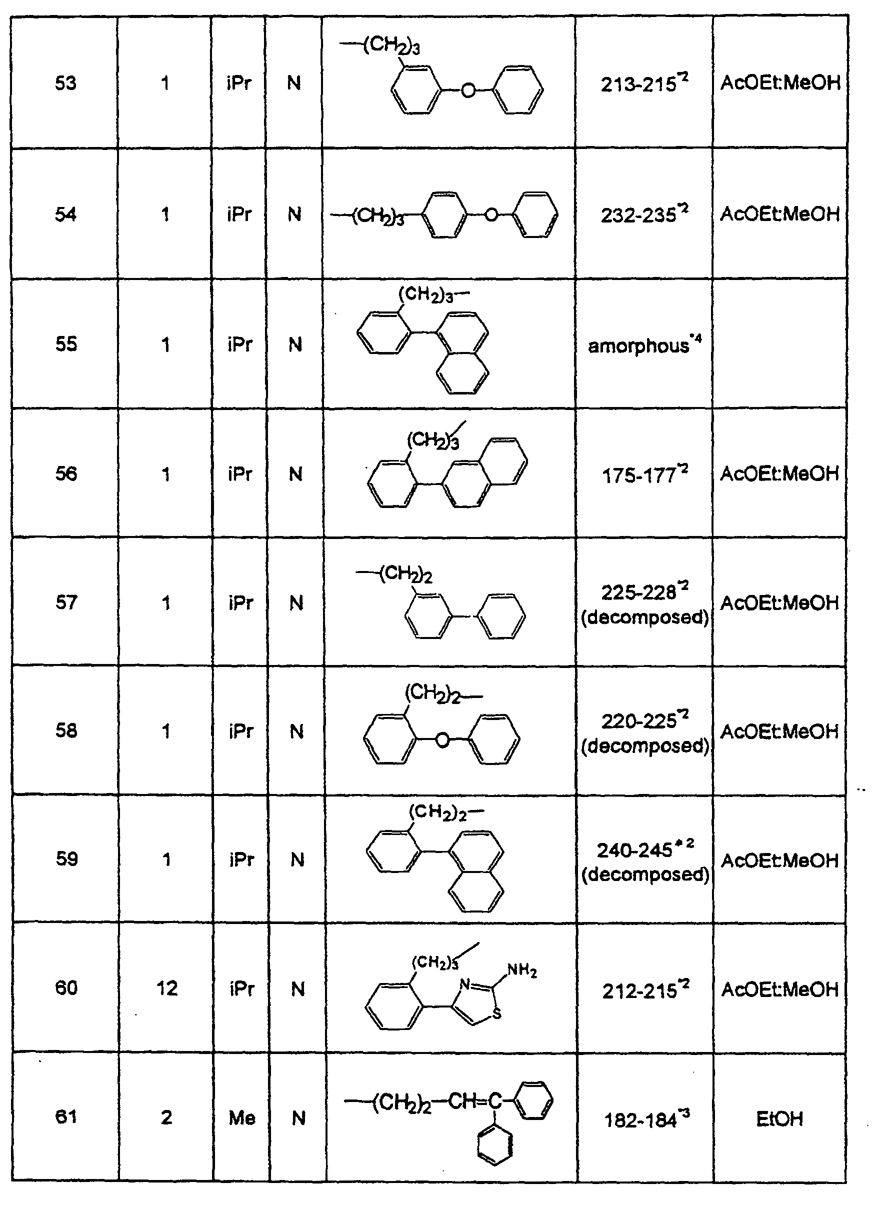

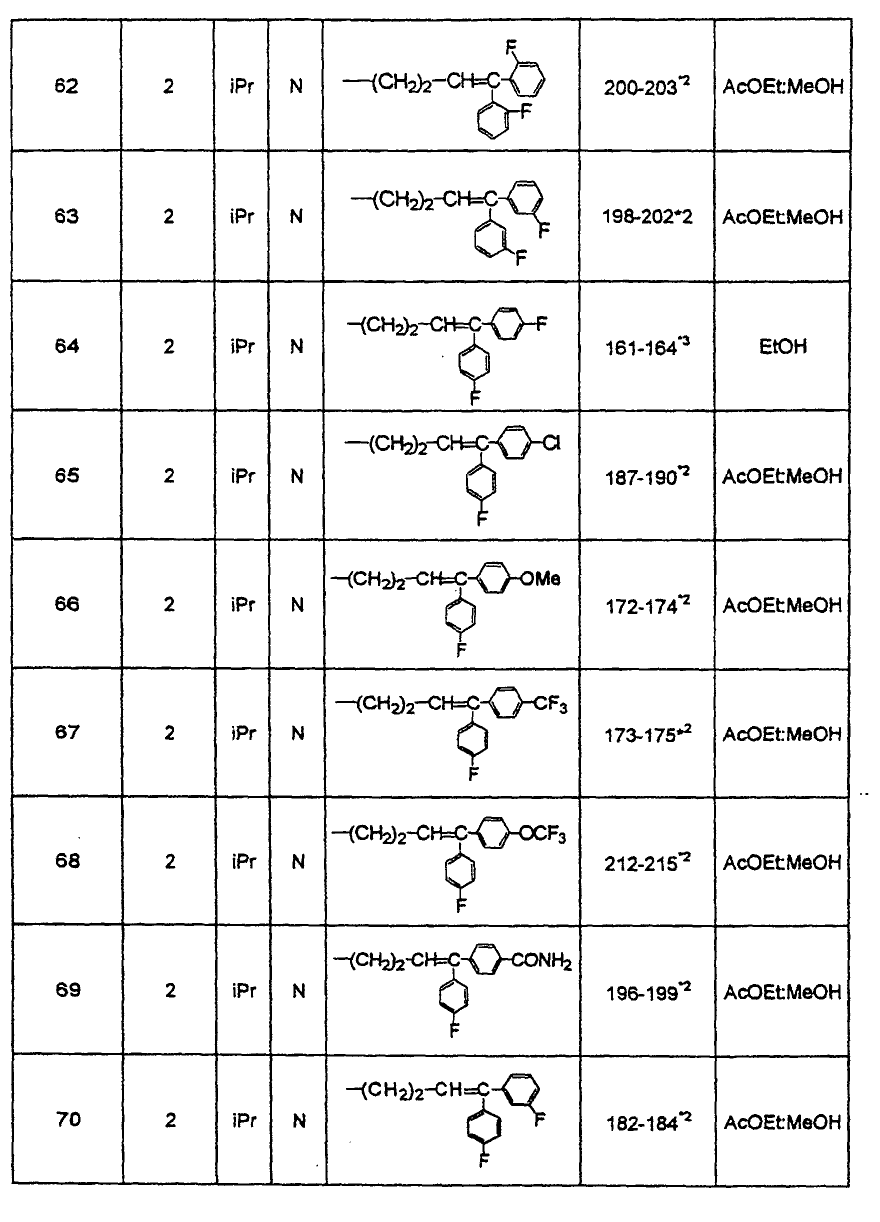

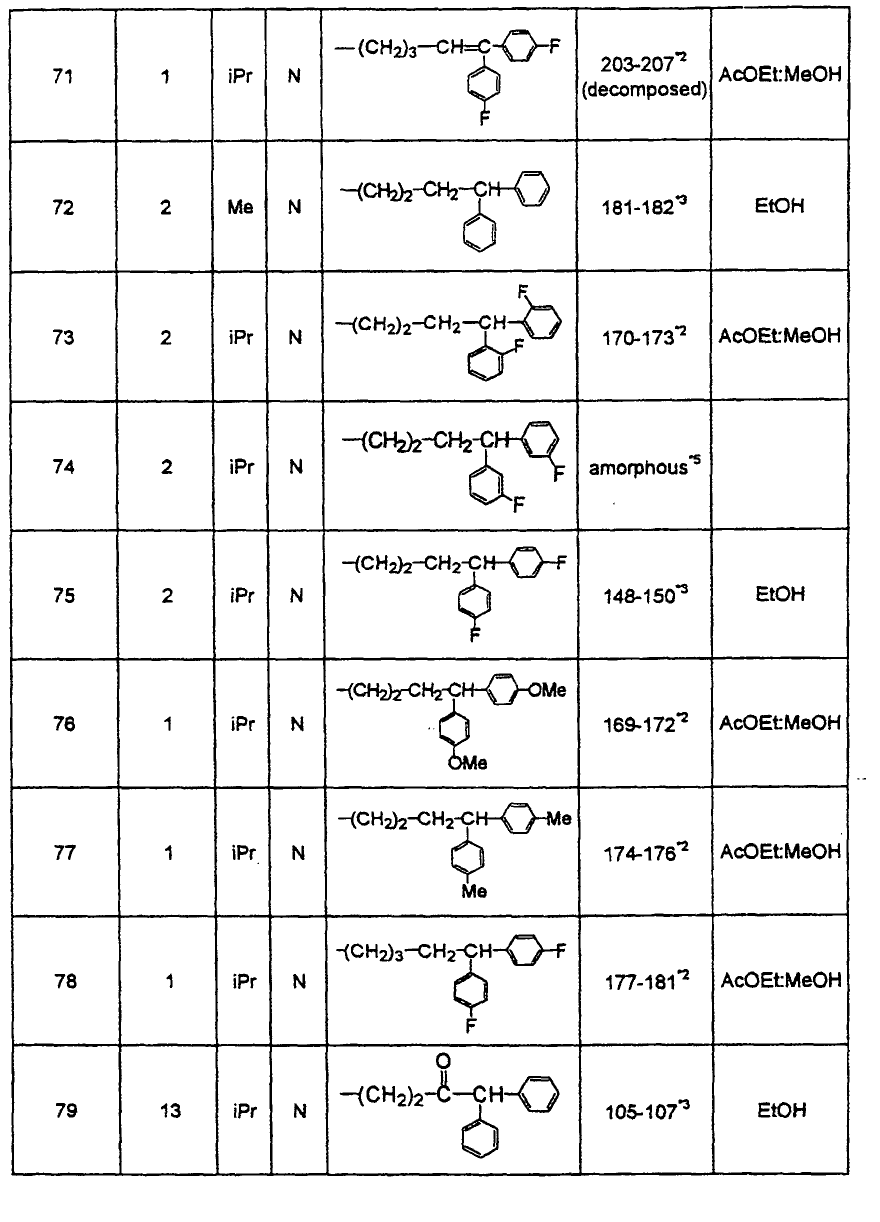

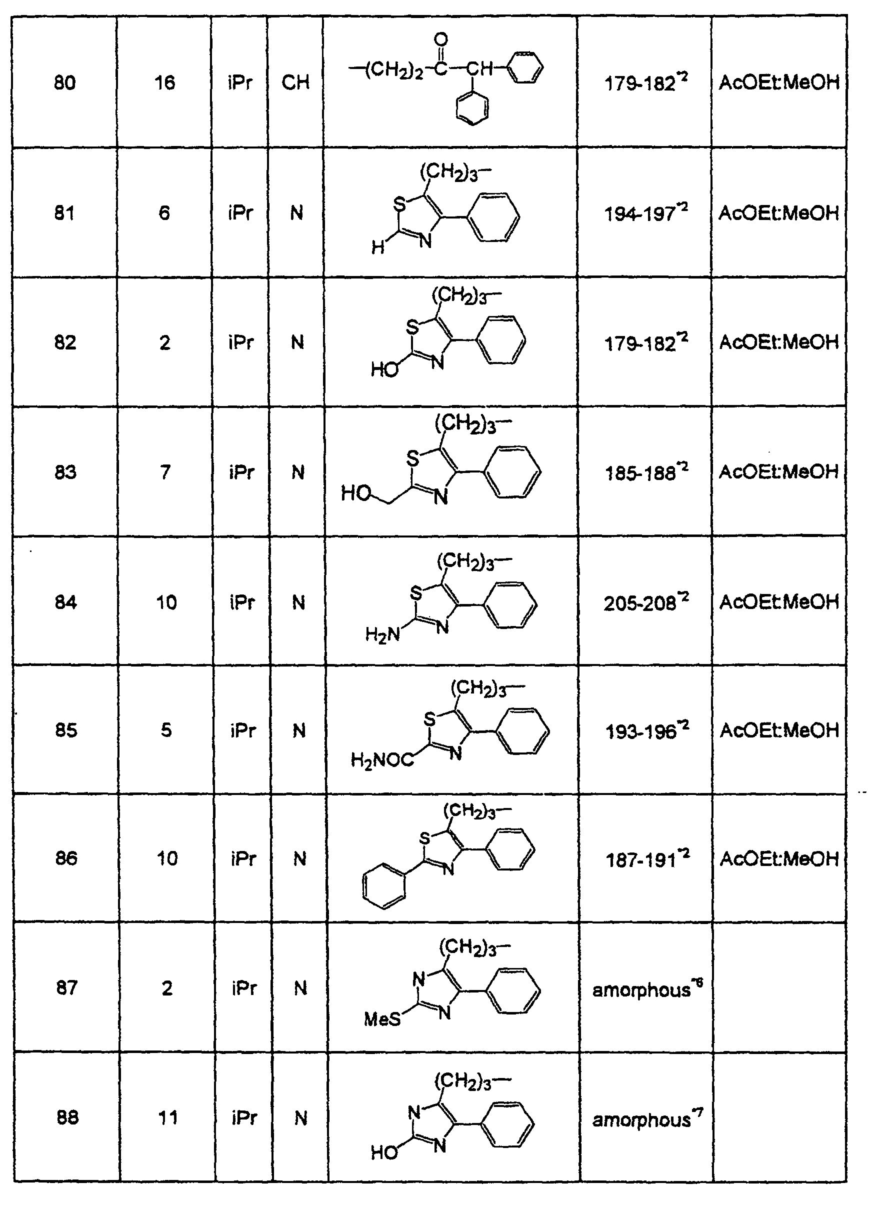

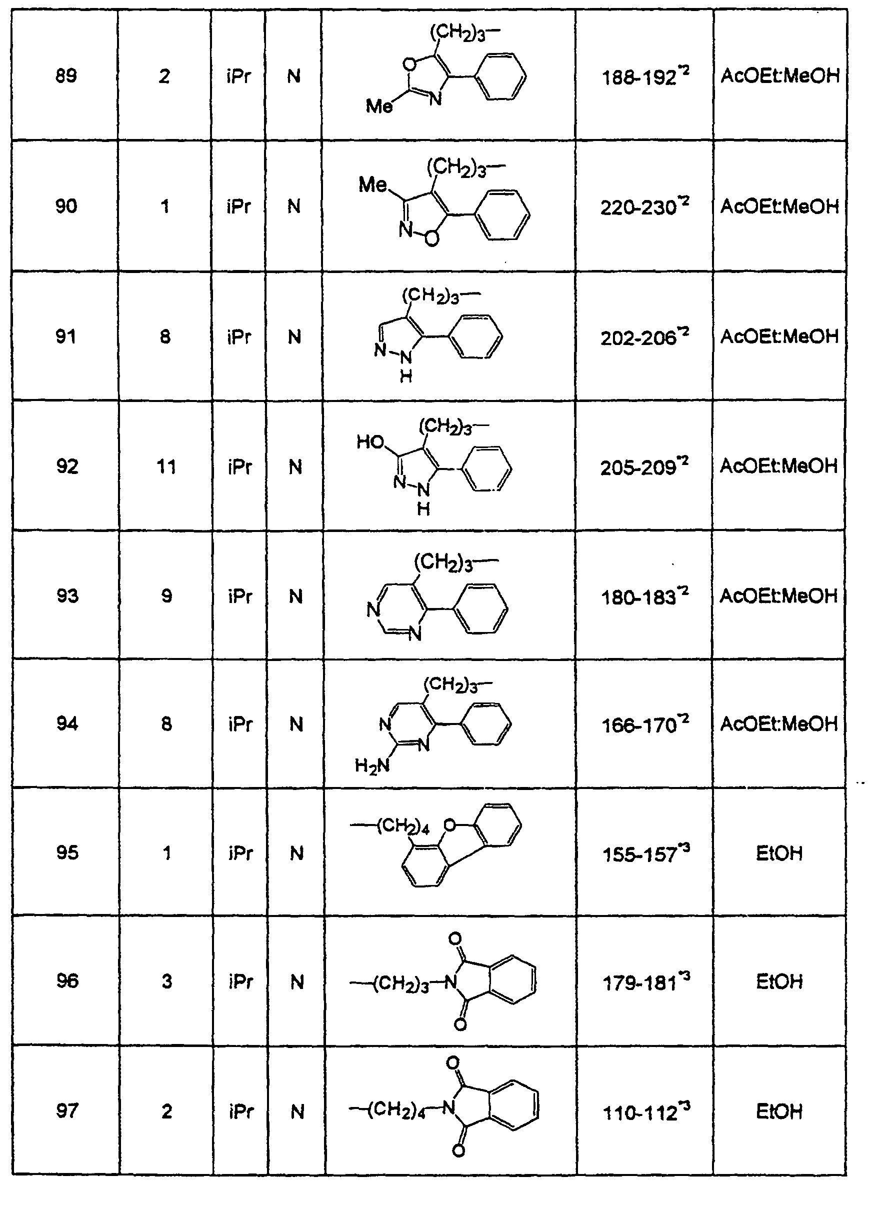

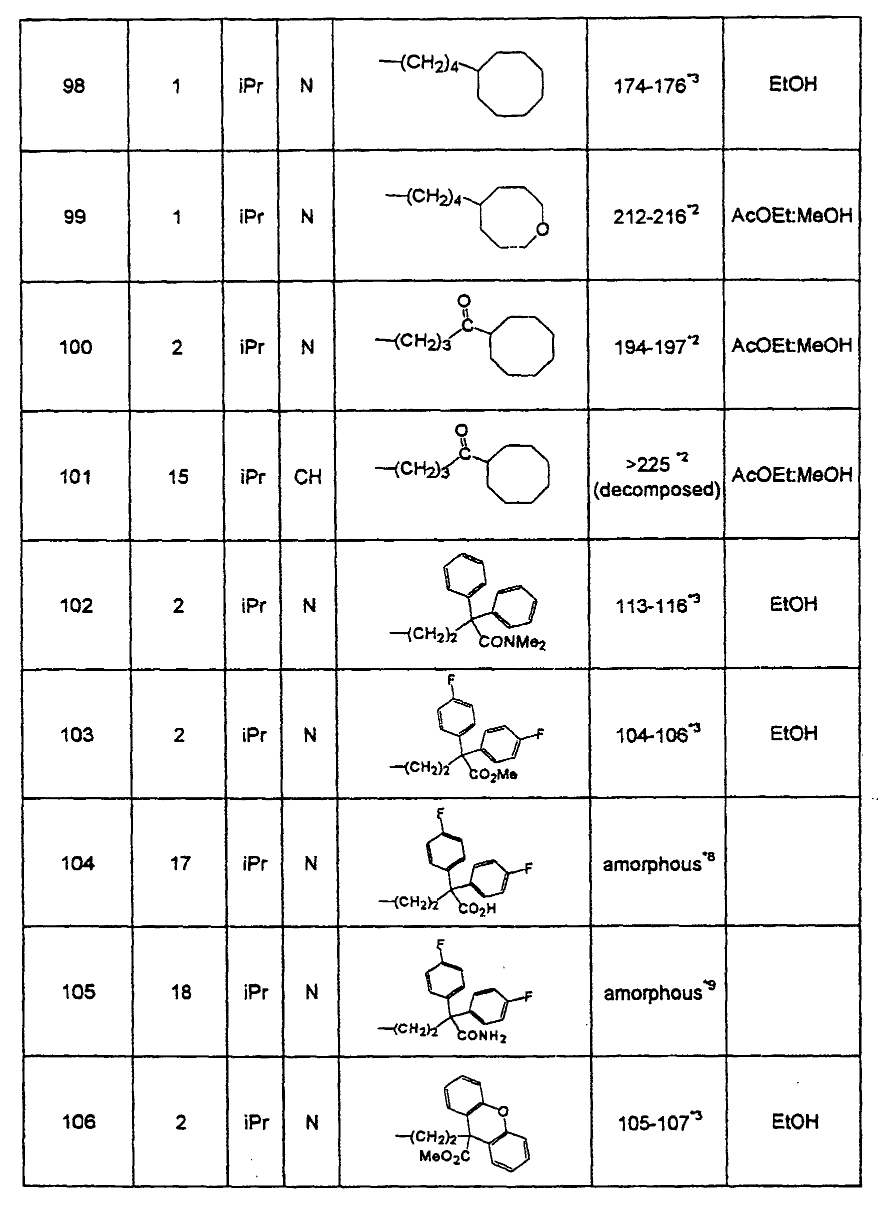

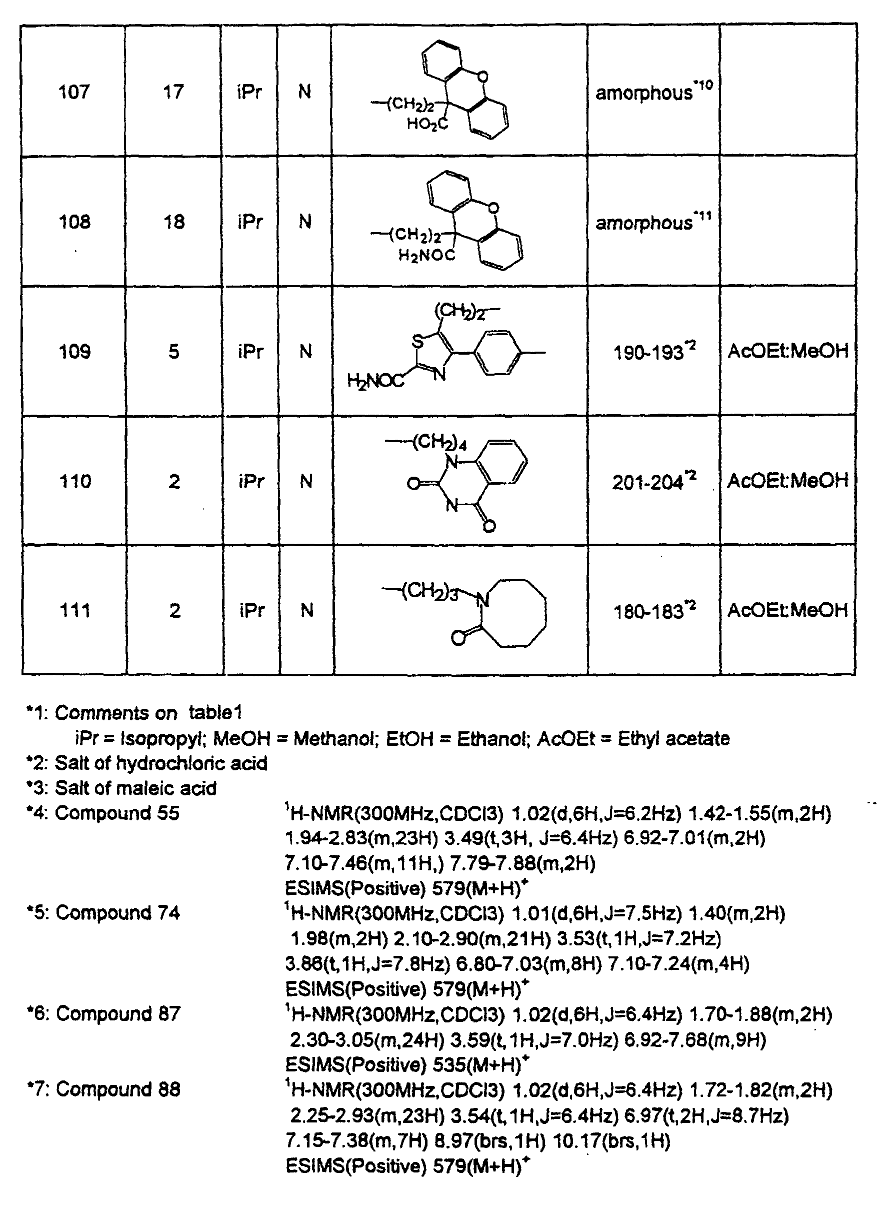

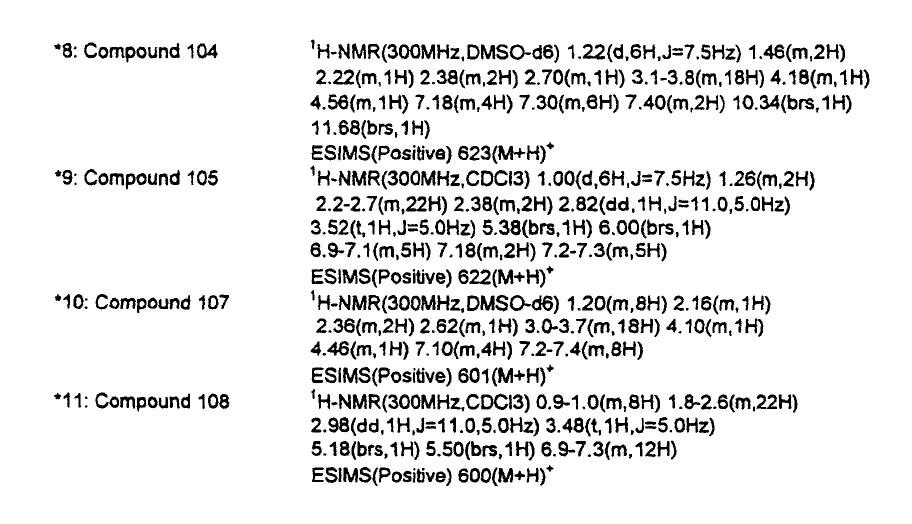

### Test Example

### (Test for binding to MC4 receptor)

The test for binding to MC4 receptor was carried out according to the method as described in Pharmacology & Toxicology, 79, 161-165, 1996. HEK-293 cell membranes expressing the human MC4 receptor were purchased from BioLinks K.K. The cell membranes were homogenized in a 50mM Tris-hydrochloric buffer (pH 7.4) containing 2 mM ethylenediaminetetraacetic acid, 10 mM calcium chloride and 100 µM phenymethylsulfonyl fluoride. The homogenate was centrifuged at 48,000xg at 4 °C for 20 minutes. The precipitate obtained by centrifugation was re-homogenized in the same buffer, and then the homogenate was centrifuged at 48,000xg at 4 °C for 20 minutes. This manipulation was repeated twice. The precipitates were suspended in a 50mM Tris-hydrochloric acid buffer (pH 7.4) containing 2mM ethylenediaminetetraacetic acid, 10 mM calcium chloride, 100 µM phenymethylsulfonyl fluoride and 0.1% bovine serum albumin so as to provide a protein concentration of 100 µg/ml. The suspension was used for the binding test as a crude membrane specimen. The crude membrane specimen (0.25 ml, 25 µg protein) was allowed to react with [¹²⁵I]Nle⁴-D-Phe⁷-α-MSH (final concentration of 0.2 nM) at 25 °C for 120 minutes. After completion of the reaction, the reaction solution was suction-filtered onto a GF/C glass fiber filter paper immersed in a 50 mM Tris-hydrochloric acid buffer containing 0.5% bovine serum (pH 7.4) by means of a cell harvester for receptor binding test. Radioactivity on the filter papers was measured using a γ-counter. The binding amount in the presence of 1 µM Nle⁴-D-Phe⁷-α-MSH was defined as non-specific binding, while specific binding was defined by subtracting the non-specific binding from the total binding, i.e. the binding in the absence of 1 µM Nle⁴-D-Phe⁷-α-MSH. A drug to be tested was dissolved in a 100% DMSO solution and was added to the membrane specimen simultaneously with [¹²⁵I]Nle⁴-D-Phe⁷-α-MSH. IC₅₀ value was calculated from inhibition curve at concentrations of from 10⁻⁸ to 10⁻⁵. Consequently, Compound 86 in Table 1 showed a value of 162 nM, for example.

### Industrial Applicability

The compounds of this invention have antagonistic activity against MC4 receptors and they are useful as a therapeutic agent for depression and anxiety neurosis.

## Claims

1. A piperazine derivative represented by the following formula: 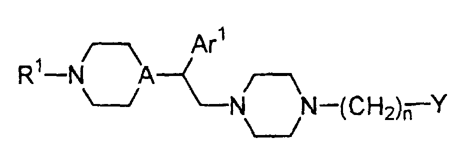 wherein n represents an integer of 1 to 8; R¹ represents a hydrogen atom or a C₁₋₁₀ alkyl group; A represents CH or a nitrogen atom; Ar¹ represents a phenyl group, or a phenyl group substituted with 1 to 3 groups arbitrarily selected from a C₁₋₁₀ alkyl group, a C₁₋₁₀ alkoxy group, an aralkyloxy group, a hydroxyl group, a halogen atom, a nitro group, an amino group, a mono- or di-substituted amino group with a C₁₋₆ alkyl group(s), a trifluoromethyl group, a trifluoromethoxy group, a cyano group, a carbamoyl group or a phenyl group; and Y is a group represented by the formula Y¹-Y²-Ar² wherein Y¹-Y² represents a single bond, an oxygen atom, C(=O), CH=CH, C(=O)-N(R²) or N(R²)-C(=O) (wherein R² represents a hydrogen atom or a C₁₋₁₀ alkyl group); and Ar² represents a phthalimido-1-yl group, a dibenzofuranyl group, a C₃₋₁₀ cycloalkyl group, a C₂₋₉ oxacycloalkyl group, a C₂₋₉ lactam-1-yl group, a 1H-quinazoline-2,4-dion-1-yl group, or a group represented by the following formula: 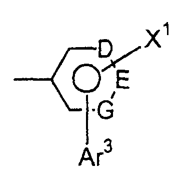 wherein D, E and G may be the same or different and each represents CH or a nitrogen atom; X¹ represents a hydrogen atom, a halogen atom, a C₁₋₁₀ alkyl group, a C₁₋₁₀ alkoxy group, a hydroxyl group, an amino group, a carbamoyl group, a C₁₋₅ alkylthio group or a phenyl group; and Ar³ represents a phenyl group, a naphthyl group, a phenoxy group, or alternatively, a phenyl, naphthyl or phenoxy group substituted with 1 to 3 groups arbitrarily selected from a C₁₋₁₀ alkyl group, a C₁₋₁₀ alkoxy group, an aralkyloxy group, a hydroxyl group, a halogen atom, a nitro group, an amino group, a mono- or di-substituted amino group with a C₁₋₅ alkyl group(s), a trifluoromethyl group, a trifluoromethoxy group, a cyano group, a carbamoyl group or a phenyl group, or a group represented by the following formula: 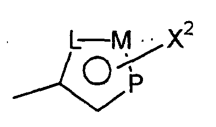 wherein L, M and P may be the same or different and each represents CH, NH, a nitrogen atom, an oxygen atom or a sulfur atom; and X² represents a hydrogen atom, a halogen atom, a C₁₋₁₀ alkyl group, a C₁₋₁₀ alkoxy group, a hydroxyl group, an amino group, a carbamoyl group, a C₁₋₅ alkylthio group or a phenyl group, or
a group represented by the following formula: 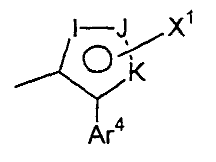 wherein I, J and K may be the same or different and each represents CH, NH, a nitrogen atom, an oxygen atom or a sulfur atom; X¹ is as previously defined; and Ar⁴ represents a phenyl group or a phenyl group substituted with 1 to groups arbitrarily selected from a C₁₋₁₀ alkyl group, a C₁₋₁₀ alkoxy group, an aralkyloxy group, a hydroxyl group, a-halogen atom, a nitro group, an amino group, a mono- or di-substituted amino group with a C₁₋₅ alkyl group(s), a trifluoromethyl group, a trifluoromethoxy group, a cyano group, a carbamoyl group or a phenyl group, or a group represented by the following formula: 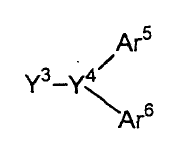 wherein Y³-Y⁴ represents CH₂-C(R^{a}) [wherein R^{a} represents a hydrogen atom or a group represented by the formula CO₂R^{b} or the formula CON(R^{b})R^{c} (wherein R^{b} and R^{c} may be the same or different and each represents a hydrogen atom or a C₁₋₁₀ alkyl group)], CH=C or C(=O)-CH; and Ar⁵ and Ar⁶ may be the same or different and each represents a phenyl group or a phenyl group substituted with 1 to 3 groups arbitrarily selected from a C₁₋₁₀ alkyl group, a C₁₋₁₀ alkoxy group, an aralkyloxy group, a hydroxyl group, a halogen atom, a nitro group, an amino group, a mono- or di-substituted amino group with a C₁₋₅ alkyl group(s), a trifluoromethyl group, a trifluoromethoxy group, a cyano group, a carbamoyl group or a phenyl group, or a group forming together with the adjacent carbon atom, a group represented by the following formula: 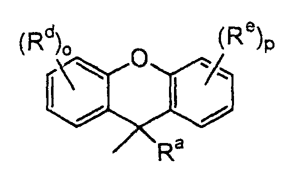 wherein R^{d} and R^{e} each represent a group arbitrarily selected from a hydrogen atom, a C₁₋₁₀ alkyl group, a C₁₋₁₀ alkoxy group, an aralkyloxy group, a hydroxyl group, a halogen atom, a nitro group, an amino group, a mono- or di-substituted amino group with a C₁₋₅ alkyl group(s), a trifluoromethyl group, a trifluoromethoxy group, a cyano group, a carbamoyl group or a phenyl group; R^{a} is as previously defined; and o and p each are an integer of 1 to 3,
or a pharmaceutically acceptable salt thereof.

2. The piperazine derivative or a pharmaceutically acceptable salt thereof according to claim 1, wherein Ar² is any of a phthalimido-1-yl group, a dibenzofuranyl group, a C₃₋₁₀ cycloalkyl group, a C₂₋₉ oxacycloalkyl group, a C₂₋₉ lactam-1-yl group, a 1H-quinazoline-2,4-dion-1-yl group or a group represented by the following formulae: 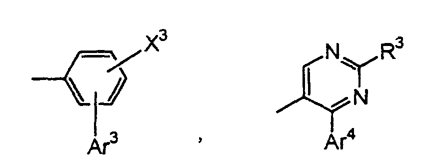 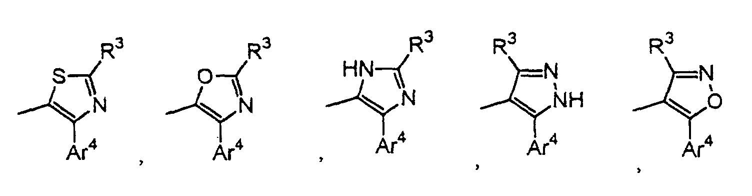 wherein Ar³ and Ar⁴ are as previously defined; R³ represents a hydrogen atom, a C₁₋₁₀ alkyl group, a hydroxyl group, an amino group, a carbamoyl group, a C₁₋₅ alkylthio group or a phenyl group; and X³ represents a hydrogen atom, a halogen atom, a C₁₋₁₀ alkyl group or a C₁₋₁₀ alkoxy group.

3. The piperazine derivative or a pharmaceutically acceptable salt thereof according to claim 2, wherein Y is a group represented by the formula Y¹-Y²-Ar² wherein Ar² is a group represented by the following formula: 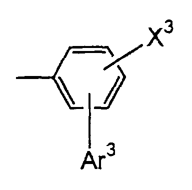 wherein X³ and Ar³ are as previously defined.

4. The piperazine derivative or a pharmaceutically acceptable salt thereof according to claim 3, wherein n is an integer of 1 to 4; R¹ is a C₁₋₄ alkyl group; Ar¹ is a phenyl group substituted with 1 to 3 halogen atoms; Y¹-Y² is a single bond, C(=O) or CH=CH; and Ar² is a group represented by the following formula: 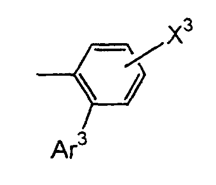 wherein Ar³ is a phenyl group, a naphthyl group, a phenoxy group or a phenyl group substituted with 1 to 3 groups arbitrarily selected from a C₁₋₁₀ alkyl group, a C₁₋₁₀ alkoxy group, a halogen atom, a nitro group, an amino group, a mono- or di-substituted amino group with a C₁₋₆ alkyl group(s), a trifluoromethyl group, a trifluoromethoxy group, a cyano group, a carbamoyl group or a phenyl group.

5. The piperazine derivative or a pharmaceutically acceptable salt thereof according to claim 3, wherein n is an integer of 1 to 3, R¹ is a C₁₋₄ alkyl group, Ar¹ is a phenyl group substituted with 1 to 3 halogen atoms, Y¹-Y² is CH₂-CH₂ or CH=CH, and Ar² is a group represented by the following formula: 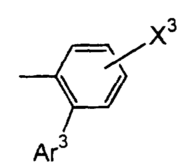 wherein Ar³ is a phenyl group, a naphthyl group, a phenoxy group or a phenyl group substituted with 1 to 3 groups arbitrarily selected from a C₁₋₁₀ alkyl group, a C₁₋₁₀ alkoxy group, a halogen atom, a nitro group, an amino group, a mono- or di-substituted amino group with a C₁₋₆ alkyl group(s), a trifluoromethyl group, a trifluoromethoxy group, a cyano group, a carbamoyl group or a phenyl group.

6. The piperazine derivative or a pharmaceutically acceptable salt thereof according to claim 3, wherein n is an integer of 2 to 5, R¹ is a C₁₋₄ alkyl group, Ar¹ is a phenyl group substituted with 1 to 3 halogen atoms, Y¹-Y² is a single bond, and Ar² is a group represented by the following formula: 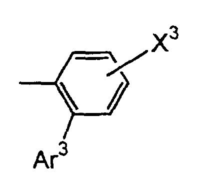 wherein Ar³ is a phenyl group, a naphthyl group, a phenoxy group or a phenyl group substituted with 1 to 3 groups arbitrarily selected from a C₁₋₁₀ alkyl group, a C₁₋₁₀ alkoxy group, a halogen atom, a nitro group, an amino group, a mono- or di-substituted amino group with a C₁₋₆ alkyl group(s), a trifluoromethyl group, a trifluoromethoxy group, a cyano group, a carbamoyl group or a phenyl group.

7. The piperazine derivative or a pharmaceutically acceptable salt thereof according to claim 3, wherein n is an integer of 1 to 3, R¹ is a C₁₋₄ alkyl group, Ar¹ is a phenyl group substituted with 1 to 3 halogen atoms, Y¹-Y² is C(=O), and Ar² is a group represented by the following formula: 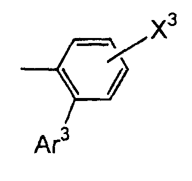 wherein Ar³ is a phenyl group or a phenyl group substituted with 1 to 3 halogen atoms.

8. The piperazine derivative or a pharmaceutically acceptable salt thereof according to claim 3, wherein n is 3, A is a nitrogen atom, R¹ is a C₁₋₄ alkyl group, Ar¹ is a phenyl group substituted with 1 to 3 halogen atoms, Y¹-Y² is a single bond, and Ar² is any of the groups represented by the following formulae: 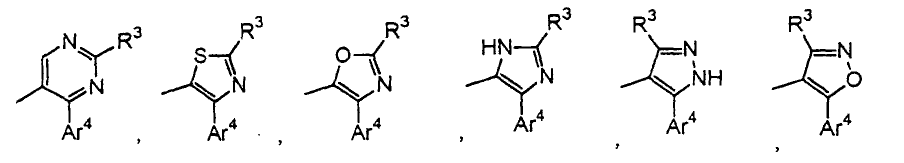 wherein Ar⁴ is a phenyl group; and R³ is a hydrogen atom, a C₁₋₁₀ alkyl group, a hydroxyl group, an amino group, a carbamoyl group, a C₁₋₅ alkylthio group or a phenyl group.

9. The piperazine derivative or a pharmaceutically acceptable salt thereof according to claim 2, wherein n is 4, R¹ is a C₁₋₄ alkyl group, A is a nitrogen atom, Ar¹ is a phenyl group substituted with 1 to 3 halogen atoms, and Y is a group represented by the group Y¹-Y²-Ar² wherein Y¹-Y² is a single bond and Ar² is a C₃₋₁₀ cycloalkyl group.

10. The piperazine derivative or a pharmaceutically acceptable salt thereof according to claim 2, wherein n is 3, R¹ is a C₁₋₄ alkyl group, A is a nitrogen atom, Ar¹ is a phenyl group substituted with 1 to 3 halogen atoms, Y is a group represented by the group Y¹-Y²-Ar² wherein Y¹-Y² is C(=O) and Ar² is a C₃₋₁₀ cycloalkyl group.

11. The piperazine derivative or a pharmaceutically acceptable salt thereof according to claim 1, wherein n is an integer of 1 to 3, R¹ is a C₁₋₄ alkyl group, A is a nitrogen atom, Ar¹ is a phenyl group substituted with 1 to 3 halogen atoms, and Y is a group represented by the following formula: 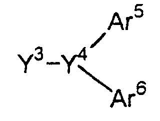 wherein Y³-Y⁴ is CH₂-CH or CH=C; and Ar⁵ and Ar⁶ may be the same or different and each is a phenyl group or a phenyl group substituted with 1 to 3 groups arbitrarily selected from a C₁₋₁₀ alkyl group, a C₁₋₁₀ alkoxy group, a halogen atom, a trifluoromethyl group, a trifluoromethoxy group or a carbamoyl group.

12. The piperazine derivative or a pharmaceutically acceptable salt thereof according to claim 1, wherein n is 2, R¹ is a C₁₋₄ alkyl group, A is a nitrogen atom, Ar¹ is a phenyl group substituted with 1 to 3 halogen atoms, and Y is a group represented by the following formula: 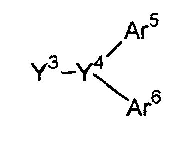 wherein Y³-Y⁴ is C(=O)-CH; and Ar⁵ and Ar⁶ may be the same or different and each is a phenyl group or a phenyl group substituted with 1 to 3 halogen atoms.

13. Use of the piperazine derivative or a pharmaceutically acceptable salt thereof according to any of claims 1-12 as an MC4 receptor antagonist.

14. A therapeutic agent for anxiety neurosis or depression which comprises as an active ingredient, the piperazine derivative or a pharmaceutically acceptable salt thereof according to any of claims 1-12.
